# EUROPEAN PATENT APPLICATION

(11) **EP 2 272 509 A1**
(43) Date of publication of application: **12.01.2011**
(21) Application number: 10172443.3
(22) Date of filing: 10.09.2007
(51) Int. Cl.: A61K 31/035

(54) **New Uses of metabotropic glutamate receptors**

(30) Priority: 11.09.2006 EP 06120424; 06.12.2006 US 868814 P; 06.12.2006 US 868805 P; 17.01.2007 US 885255 P
(62) Divisional of application: 07818088.2
(71) Applicant: Novartis AG, 4056 Basel (CH)
(72) Inventor: Glatthar, Ralf, 79713, Bad Sackingen (DE); Johns, Donald, Woburn, MA 01801 (US); Umbricht, Daniel, 8037, Zurich (CH)
(74) Representative: Rouquayrol, Céline Hélène

(57) **Abstract**

The invention concerns the use an mGluR modulator, e.g. an mGluR5 modulator, for the treatment, prevention or delay of progression of cognitive dysfunction.

## Description

The present invention relates to new pharmaceutical uses of compounds acting as modulators of metabotropic glutamate receptors ("mGluR modulators"), including antagonists of metabotropic glutamate receptors ("mGluR antagonists"). In particular, the present invention relates new uses of antagonists of metabotropic glutamate type-5 receptors ("mGluR5 antagonists").

WO 2005/079802, WO 2003/047581, WO 2004/000316, WO 2005/044265, WO 2005/044266, WO 2005/044267, WO 2006/114262 and WO 2007/071358 disclose mGluR5 antagonists and their use as pharmaceuticals.

It has been surprisingly found that compounds having mGluR modulating activity, in particular antagonistic activity, may be used to treat cognitive dysfunction. In particular, it has been found that mGluR5 modulators, e.g. mGluR5 antagonists, may be used to treat cognitive dysfunction.

Accordingly, a first aspect of the invention concerns the use of an mGluR modulator for the treatment, prevention and/or delay of progression of cognitive dysfunction.

A further aspect of the invention relates to a method for the treatment, prevention or delay of progression of cognitive dysfunction in a subject in need of such treatment, which comprises administering to said subject a therapeutically effective amount of an mGluR, e.g. mGluR5, modulator.

A further aspect of the invention relates to a pharmaceutical composition comprising an mGluR, e.g. mGluR5, modulator for the treatment, prevention or delay of progression of cognitive dysfunction.

A further aspect of the invention relates to the use of an mGluR, e.g. mGluR5, modulator for the manufacture of a medicament for the treatment, prevention or delay of progression of cognitive dysfunction.

The mGluR modulator may be an mGluR5 modulator. In certain embodiments, the mGluR modulator is an mGluR, e.g. mGluR5, antagonist.

In the present specification, the following definitions shall apply if no specific other definition is given:
"Alkyl" represents a straight-chain or branched-chain alkyl group, preferably represents a straight-chain or branched-chain C₁₋₁₂ alkyl, particularly preferably represents a straight-chain or branched-chain C₁₋₆ alkyl; for example, methyl, ethyl, n- or iso-propyl, n-, iso-, sec- or tert-butyl, n-pentyl, n-hexyl, n-heptyl, n-octyl, n-nonyl, n-decyl, n-undecyl, n-dodecyl, with particular preference given to methyl, ethyl, n-propyl and iso-propyl.
"Alkandiyl" represents a straight-chain or branched-chain alkandiyl group bound by two different carbon atoms to the molecule, it preferably represents a straight-chain or branched-chain C₁₋₁₂ alkandiyl, particularly preferably represents a straight-chain or branched-chain C₁₋₆ alkandiyl; for example, methandiyl (-CH₂-), 1,2-ethanediyl (-CH₂-CH₂-), 1,1-ethanediyl ((-CH(CH₃)-), 1,1-, 1,2-, 1,3-propanediyl and 1,1-, 1,2-, 1,3-, 1,4-butanediyl, with particular preference given to methandiyl, 1,1-ethanediyl, 1,2-ethanediyl, 1,3-propanediyl or 1,4-butanediyl.
Each alkyl part of "alkoxy", "alkoxyalkyl", "alkoxycarbonyl", "alkoxycarbonylalkyl" and "halogenalkyl" shall have the same meaning as described in the above-mentioned definition of "alkyl".
"Alkenyl" represents a straight-chain or branched-chain alkenyl group, preferably C₂₋₆ alkenyl, for example, vinyl, allyl, 1-propenyl, isopropenyl, 2-butenyl, 2-pentenyl, 2-hexenyl, etc. and preferably represents C₂₋₄ alkenyl.
"Alkendiyl" represents a straight-chain or branched-chain alkendiyl group bound by two different carbon atoms to the molecule, it preferably represents a straight-chain or branched-chain C₂₋₆ alkandiyl; for example, -CH=CH-, -CH=C(CH₃)-, -CH=CH-CH₂-, -C(CH₃)=CH-CH₂-, -CH=C(CH₃)-CH₂-, -CH=CH-C(CH₃)H-, -CH=CH-CH=CH-, -C(CH₃)=CH-CH=CH-, -CH=C(CH₃)-CH=CH-, with particular preference given to -CH=CH-CH₂-, -CH=CH-CH=CH-.
"Alkynyl" represents a straight-chain or branched-chain alkynyl group, preferably C₂₋₆ alkynyl, for example, ethenyl, propargyl, 1-propynyl, isopropenyl, 1- (2- or 3) butynyl, 1- (2- or 3) pentenyl, 1- (2- or 3) hexenyl, etc. ,preferably represents C₂₋₄ alkynyl and particularly preferably represents ethynyl.
"Aryl" represents an aromatic hydrocarbon group, preferably a C₆₋₁₀ aromatic hydrocarbon group; for example phenyl, naphthyl, especially phenyl.
"Aralkyl" denotes an "aryl" bound to an "alkyl" (both as defined above) an represents, for example benzyl, α-methylbenzyl, 2-phenylethyl, α,α-dimethylbenzyl, especially benzyl.
"Heterocycle" represents a saturated, partly saturated or aromatic ring system containing at least one hetero atom. Preferably, heterocycles consist of 3 to 11 ring atoms of which 1-3 ring atoms are hetero atoms. Heterocycles may be present as a single ring system or as bicyclic or tricyclic ring systems; preferably as single ring system or as benz-annelated ring system. Bicyclic or tricyclic ring systems may be formed by annelation of two or more rings, by a bridging atom, e.g. oxygen, sulfur, nitrogen or by a bridging group, e.g. alkandiyl or alkenediyl. A heterocycle may be substituted by one or more substituents selected from the group consisting of oxo (=O), halogen, nitro, cyano, alkyl, alkandiyl, alkenediyl, alkoxy, alkoxyalkyl, alkoxycarbonyl, alkoxycarbonylalkyl, halogenalkyl, aryl, aryloxy and arylalkyl. Examples of heterocyclic moieties include pyrrole, pyrroline, pyrrolidine, pyrazole, pyrazoline, pyrazolidine, imidazole, imidazoline, imidazolidine, triazole, triazoline, triazolidine, tetrazole, furane, dihydrofurane, tetrahydrofurane, furazane (oxadiazole), dioxolane, thiophene, dihydrothiophene, tetrahydrothiophene, oxazole, oxazoline, oxazolidine, isoxazole, isoxazoline, isoxazolidine, thiazole, thiazoline, thiazlolidine, isothiazole, istothiazoline, isothiazolidine, thiadiazole, thiadiazoline, thiadiazolidine, pyridine, piperidine, pyridazine, pyrazine, piperazine, triazine, pyrane, tetrahydropyrane, thiopyrane, tetrahydrothiopyrane, oxazine, thiazine, dioxine, morpholine, purine, pterine, and the corresponding benz-annelated heterocycles, e.g. indole, isoindole, cumarine, cumaronecinoline, isochinoline, cinnoline and the like.
"Hetero atoms" are atoms other than carbon and hydrogen, preferably nitrogen (N), oxygen (O) or sulfur (S).
"Halogen" represents fluoro, chloro, bromo or iodo, preferably represents fluoro, chloro or bromo and particularly preferably represents chloro.

Various compounds having mGluR, in particular mGluR5, modulating activity are described herein. Where the specification refers to compounds, agents or active ingredients of the invention, this is generally taken to mean a compound having mGluR modulating activity unless specified otherwise.

Compounds of the invention may exist in free or acid addition salt form. In this specification, unless otherwise indicated, reference to "compounds of the invention" is to be understood as embracing the compounds in any form, for example free base or acid addition salt form. Salts which are unsuitable for pharmaceutical uses but which can be employed, for example, for the isolation or purification of free compounds of the invention, such as picrates or perchlorates, are also included. For therapeutic use, only pharmaceutically acceptable salts or free compounds are employed (where applicable in the form of pharmaceutical preparations), and are therefore preferred.

It will be understood that any discussion of methods or references to the active ingredients also includes pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. The active ingredient or a pharmaceutically acceptable salt thereof may also be used in the form of a hydrate or may include other solvents used for crystallization. Examples of mGluR5 modulators, e.g. antagonists, and their manufacture are known, e.g. from WO 03/047581 and WO 2006/114262, both of which are incorporated herein by reference.

On account of the asymmetrical carbon atom(s) that may be present in the compounds of the invention and their salts, the compounds may exist in optically active form or in form of mixtures of optical isomers, e.g. in form of racemic mixtures or diastereomeric mixtures. All optical isomers and their mixtures, including racemic mixtures, are part of the present invention.

In one embodiment, the mGluR modulator is a compound of the formula (I), for example as described in WP 2007/071358: 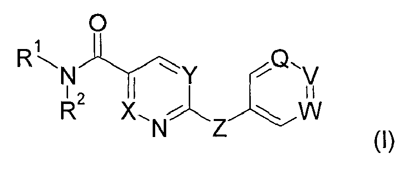 wherein
- **R¹**: represents optionally substituted alkyl or optionally substituted benzyl; and
- **R²**: represents hydrogen (H), optionally substituted alkyl or optionally substituted benzyl; or
- **R¹** and **R²**: form together with the nitrogen atom to which they are attached an optionally substituted heterocycle with less than 14 ring atoms;
- **R³**: represents halogen, alkyl, alkoxy, alkylamino or dialkylamino;
- **R⁴**: represents hydroxy (OH), halogen, alkyl or alkoxy;
- **Q**: represents CH, CR⁴ or N;
- **V**: represents CH, CR⁴ or N;
- **W**: represents CH, CR⁴ or N;
- **X**: represents CH or N;
- **Y**: represents CH, CR³ or N;
- **Z**: represents CH₂, NH or O; and
provided that Q, V and W are not N at the same time;
in free base or acid addition salt form.

In another embodiment, the mGluR modulator is a compound of the formula (II), wherein a compound of the formula (II) is a compound of formula (I) in which at least one of Q, V and W is N; in free base or acid addition salt form.

In yet a further embodiment, the mGluR modulator is a compound of the formula (III), wherein a compound of the formula (III) is a compound of formula (II) in which Y is CR³; in free base or acid addition salt form.

Preferred substituents, preferred ranges of numerical values or preferred ranges of the radicals present in the formula (I), (II) and (III) and the corresponding intermediate compounds are defined below.
- X: preferably represents CH.
- Y: preferably represents CH or CR³, wherein R³ preferably represents halogen, particular preferably chloro.
- Z: preferably represents NH.
- R³: preferably represents fluoro, chloro, C₁₋₄ alkyl, e.g. methyl.
- R³: particularly preferably represents chloro.
- R¹ and R²: preferably form together with the nitrogen atom to which they are attached an unsubstituted or substituted heterocycle having 3 - 11 ring atoms and 1 - 4 hetero atoms; the hetero atoms being selected from the group consisting of N, O, S, the substituents being selected from the group consisting of oxo (=O), hydroxy, halogen, amino, nitro, cyano, C₁₋₄ alkyl, C₁₋₄ alkoxy, C₁₋₄ alkoxyalkyl, C₁₋₄ alkoxycarbonyl, C₁₋₄ alkoxycarbonylalkyl, C₁₋₄ halogenalkyl, C₆₋₁₀ aryl, halogen- C₆₋₁₀ aryl, C₆₋₁₀ aryloxy and C₆₋₁₀-aryl-C₁₋₄ alkyl.
- R¹ and R²: form together with the nitrogen atom to which they are attached form an unsubstituted, a single or twofold substituted heterocycle having 5 - 9 ring atoms and 1 - 3 hetero atoms; the hetero atoms being selected from the group consisting of N and O; the substituents being selected from the group consisting of halogen and C₁₋₄ alkyl.
- R¹ and R²: preferably form together with the nitrogen atom to which they are attached an unsubstituted, a single or twofold substituted heterocycle selected from the group consisting of 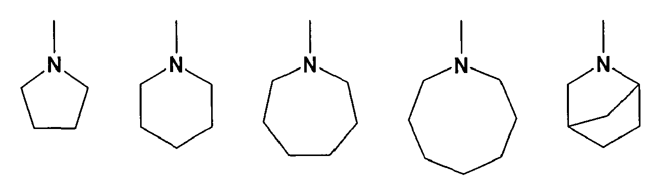 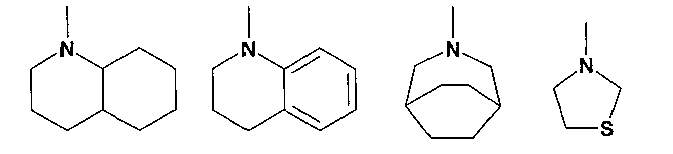 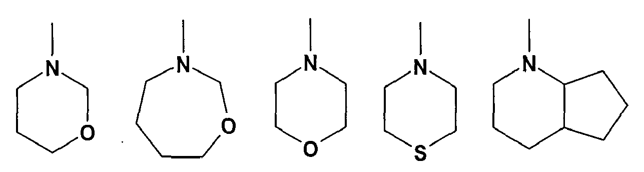 and the substituents being selected from the group consisting of fluoro, chloro, methyl, ethyl, propyl, butyl, trifluoromethyl, fluoropropyl and difluoropropyl.
- R¹ and R²: preferably represent, independently from each other, C₁-C₄ alkyl or benzyl, optionally substituted by C₁-C₄ alkoxy or halogen.

The above mentioned general or preferred radical definitions apply both to the end products of the formulae (I), (II) and (III) and also, correspondingly, to the starting materials or intermediates required in each case for the preparation. These radical definitions can be combined with one another at will, i.e. including combinations between the given preferred ranges. Further, individual definitions may not apply.

Preference according to the invention is given to compounds of the formulae (I), (II) and (III) which contain a combination of the meanings mentioned above as being preferred.

Particular preference according to the invention is given to compounds of the formulae (I), (II) and (III) which contain a combination of the meanings listed above as being particularly preferred.

Very particular preference according to the invention is given to the compounds of the formula (I) which contain a combination of the meanings listed above as being very particularly preferred.

Preferred are those compounds of formulae (I), (II) and (III) wherein R² represents an unsubstituted or substituted heterocycle.

Particular preferred are compounds of formulae (IIa to IIe) as shown below: 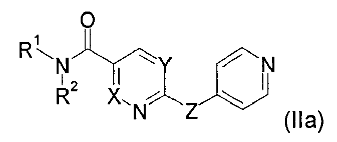 wherein the substituents have the meaning given in this specification; 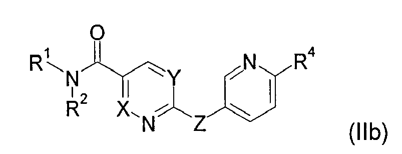 wherein the substituents have the meaning given in this specification; 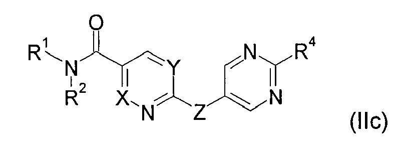 wherein the substituents have the meaning given in this specification; 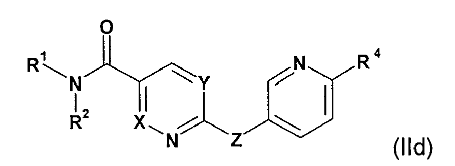 wherein R⁴ represents C₁-C₄ alkyl, preferably methyl, and the other substituents have the meaning given in this specification; 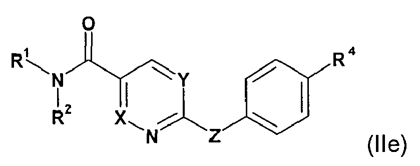 wherein R⁴ represents halogen, preferably chloro, and the other substituents have the meaning given in this specification.

Further preferred compounds of the present invention have the formulae (IIIa to IIIe) as shown below: 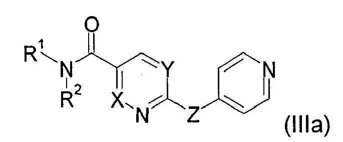 wherein all of the substituents have the meaning given in this specification; 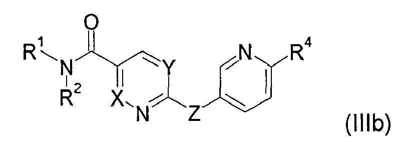 wherein the substituents have the meaning given in this specification; 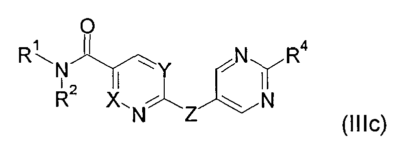 wherein the substituents have the meaning given in this specification; 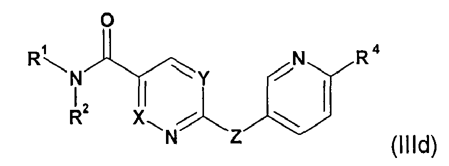 wherein R⁴ represents C₁-C₄ alkyl, preferably methyl, and the other substituents have the meaning given in this specification; 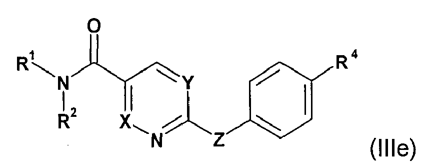 wherein R⁴ represents halogen, preferably chloro, and the other substituents have the meaning given in this specification.

Particular compounds of the formulae (I), (II) and (III) include those described in the Examples given herein.

In another embodiment, the mGluR modulator is a compound of the formula (IV), for example as described in WO 03/047581: 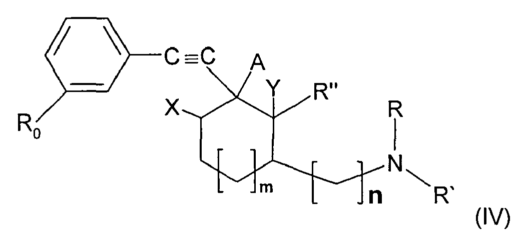 wherein
m is 0 or 1,
n is 0 or 1 and
A is hydroxy
X is hydrogen and
Y is hydrogen, or
A forms a single bond with X or with Y;
R₀ is hydrogen, (C₁₋₄)alkyl, (C₁₋₄)alkoxy, trifluoromethyl, halogen, cyano, nitro, -COOR₁ wherein R₁ is (C₁₋₄)alkyl or -COR₂ wherein R₂ is hydrogen or (C₁₋₄)alkyl, and
R is -COR₃, -COOR₃, -CONR₄R₅ or -SO₂R₆, wherein R₃ is (C₁₋₄)alkyl, (C₃₋₇)cycloalkyl or optionally substituted phenyl, 2-pyridyl or 2-thienyl; R₄ and R₅, independently, are hydrogen or (C₁₋₄)alkyl; and R₆ is (C₁₋₄)alkyl, (C₃₋₇)cycloalkyl or optionally substituted phenyl,
R' is hydrogen or (C₁₋₄)alkyl and
R" is hydrogen or (C₁₋₄)alkyl, or
R' and R" together form a group -CH₂-(CH₂)ₘ-
wherein m is 0, 1 or 2, in which case one of n and m is different from 0,
with the proviso that R₀ is different from hydrogen, trifluoromethyl and methoxy when n is 0, A is hydroxy, X and Y are both hydrogen, R is COOEt and R' and R" together form a group - (CH₂)₂-,
in free base or acid addition salt form.

Exemplary compounds of formula (IV) include:
(-)-(3aR,4S,7aR)-4-Hydroxy-4-m-tolylethynyl-octahydro-indole-1-carboxylic acid methyl ester
(-)-(3aR,4S,7aR)-4-Hydroxy-4-m-tolylethynyl-octahydro-indole-1-carboxylic acid ethyl ester
(-)-(3aR,4S,7aR)-Furan-2-yl-(4-hydroxy-4-m-tolylethynyl-octahydro-indol-1-yl)-methanone
(±)- (3aRS,4SR,7aRS)-4-(3-Chlorophenylethynyl)-4-hydroxy-octahydro-indole-1-carboxylic acid ethyl ester
(±)-(3aRS,4SR,7aRS)-4-(3-Fluoro-phenylethynyl)-4-hydroxy-octahydro-indole-1-carboxylic acid ethyl ester
(3aRS,4SR,7aRS)-4-Hydroxy-4-phenylethynyl-octahydro-indole-1-carboxylic acid(S)(tetrahydrofuran-3-yl)ester
(3aRS,4SR,7aRS)-4-Hydroxy-4-phenylethynyl-octahydro-indole-1-carboxylic acid(R)(tetrahydrofuran-3-yl)ester
(3aRS,4SR,7aRS)-4-Hydroxy-4-(3-chlorophenylethynyl)-octahydro-indol-1-carboxylic acid-(S)(tetrahydrofuran-3yl)ester
(±)-(3aRS,4SR,7aRS)-4-Hydroxy-4-m-tolylethynyl-octahydro-indole-1-carboxylic acid ethyl ester
(±)-(3aRS,4SR,7aRS)-4-(4-Fluoro-phenylethynyl)-4-hydroxy-octahydro-indole-1-carboxylic acid ethyl ester
(±)-(3aRS,4SR,7aRS)-4-(3-chlorophenylethynyl)-4-hydroxy-1-methanesulfonyl-octahydroindole
(±)-(3aRS,7aRS)-4-Phenylethynyl-2,3,3a,6,7,7a-hexahydro-indole-1-carboxylic acid ethyl ester and (±)-(RS)-4-phenylethynyl-2,3,5,6,7,7a-hexahydro-indole-1-carboxylic acid ethyl ester
(±)-(3RS,7aRS)-2,2,2-Trifluoro-1-(4-phenylethynyl-2,3,3a,6,7,7a-hexahydro-indol-1-yl)-ethanone
(±)-(RS)-4-m-Tolylethynyl-2,3,5,6,7,7a-hexahydro-indole-1-carboxylic acid ethyl ester
(±)-(3RS,7aRS)-4-*m*-Tolylethynyl-2,3,3a,6,7,7a-hexahydro-indole-1-carboxylic acid ethyl ester
(±)-(3RS,7aRS)-4-(4-Chloro-phenylethynyl)-2,3,3a,6,7,7a-hexahydro-indole-1-carboxylic acid ethyl ester
(±)-(3RS,7aRS)-4-(2-Fluoro-phenylethynyl)-2,3,3a,6,7,7a-hexahydro-indole-1-carboxylic acid ethyl ester
(±)-(3RS,7aRS)-4-(3-Fluoro-phenylethynyl)-2,3,3a,6,7,7a-hexahydro-indole-1-carboxylic acid ethyl ester
(±)-(RS)-4-(3-Fluoro-phenylethynyl)-2,3,5,6,7,7a-hexahydro-indole-1-carboxylic acid ethyl ester
(±)-(3RS,7aRS)- 4-(3-Methoxy-phenylethynyl)-2,3,3a,6,7,7a-hexahydro-indole-1-carboxylic acid ethyl ester
(±)-(RS)-4-(3-Methoxy-phenylethynyl)-2,3,5,6,7,7a-hexahydro-indole-1-carboxylic acid ethyl ester
(±)-(3aRS,4RS,7aSR)-4-Hydroxy-4-phenylethynyl-octahydro-isoindole-2-carboxylic acid ethyl ester
(±)-(3aRS,4RS,7aSR)-4-Hydroxy-4-m-tolylethynyl-octahydro-isoindole-2-carboxylic acid ethyl ester
(±)-(3aRS,4RS,7aSR)-4-Hydroxy-4-p-tolylethynyl-octahydro-isoindole-2-carboxylic acid ethyl ester
(±)-(3aRS,4RS,7aSR)-4-(3-Cyano-phenylethynyl)-4-hydroxy-octahydro-isoindole-2-carboxylic acid ethyl ester
(±)-(3aRS,4RS,7aSR)-4-Hydroxy-4-(3-methoxy-phenylethynyl)-octahydro-isoindole-2-carboxylic acid ethyl ester
(±)-(3aRS,4RS,7aSR)-4-(3-Fluoro-phenylethynyl)-4-hydroxy-octahydro-isoindole-2-carboxylic acid ethyl ester
(±)-(3aRS,4RS,7aSR)-4-Hydroxy-4-phenylethynyl-octahydro-isoindole-2-carboxylic acid *tert-*butyl ester
(±)-(3aRS,4RS,7aSR)-4-Hydroxy-4-m-tolylethynyl-octahydro-isoindole-2-carboxylic acid *tert-*butyl ester
(±)-(3aRS,4RS,7aSR)-4-Hydroxy-4-m-tolylethynyl-octahydro-isoindole-2-carboxylic acid methyl ester
(±)-(3aRS,4RS,7aSR)-Furan-2-yl-(4-hydroxy-4-m-tolylethynyl-octahydro-isoindol-2-yl)-methanone
(±)-(3aRS,4RS,7aSR)-Cyclopropyl-(4-hydroxy-4-m-tolylethynyl-octahydro-isoindol-2-yl)-methanone
(±)-(3aRS,4RS,7aSR)- (4-Hydroxy-4-m-tolylethynyl-octahydro-isoindol-2-yl)-pyridin-3-yl-methanone
(±)-((1SR,3SR)-3-Hydroxy-3-*m*-tolylethynyl-cyclohexyl)-methyl-carbamic acid methyl ester and (±)-((1RS,3SR)-3-hydroxy-3-m-tolylethynyl-cyclohexyl)-methyl-carbamic acid methyl ester
(±)-(1RS,3SR)-((3-Hydroxy-3-*m*-tolylethynyl-cyclohexyl)-(4-methoxy-benzyl)-carbamic acid ethyl ester
(±)-(1RS,3RS)-((3-Hydroxy-3-*m*-tolylethynyl-cyclohexyl)-(4-methoxy-benzyl)-carbamic acid ethyl ester
(±)-[(1RS,3SR)-3-Hydroxy-3-(3-methoxy-phenylethynyl)-5,5-dimethyl-cyclohexyl]-methyl-carbamic acid methyl ester
(±)-(1RS,3SR)-(3-Hydroxy-5,5-dimethyl-3-*m*-tolylethynyl-cyclohexyl)-methyl-carbamic acid methyl ester
(±)-[(1RS,3SR)-3-(3-Fluoro-phenylethynyl)-3-hydroxy-5,5-dimethyl-cyclohexyl]-methyl-carbamic acid methyl ester
(±)-[(1RS,3RS)-3-(3-Fluoro-phenylethynyl)-3-hydroxy-cyclohexyl]-methyl-carbamic acid methyl ester
(±)-[(1RS,3SR)-3-(3-Fluoro-phenylethynyl)-3-hydroxy-cyclohexyl]-methyl-carbamic acid methyl ester
(±)-[(1RS,3RS)-3-Hydroxy-3-(3-methoxy-phenylethynyl)-cyclohexyl]-methyl-carbamic acid methyl ester
(±)-[(1RS,3SR)-3-Hydroxy-3-(3-methoxy-phenylethynyl)-cyclohexyl]-methyl-carbamic acid methyl ester
(±)-[(1RS,3RS)-3-(3-Chloro-phenylethynyl)-3-hydroxy-cyclohexyl]-methyl-carbamic acid methyl ester
(±)-[(1RS,3SR)-3-(3-Chloro-phenylethynyl)-3-hydroxy-cyclohexyl]-methyl-carbamic acid methyl ester
(±)-(1RS,3RS)-N-(3-hydroxy-3-m-tolylethynyl-cyclohexyl)-acetamide
(±)-(1RS,3SR)-N-(3-hydroxy-3-m-tolylethynyl-cyclohexyl)-acetamide
(±)-(1RS,3RS)-(3-Hydroxy-3-m-tolylethynyl-cyclohexyl)-carbamic acid ethyl ester
(±)-(1RS,3SR)-(3-Hydroxy-3-m-tolylethynyl-cyclohexyl)-carbamic acid ethyl ester
(±)-(1RS,3RS)-[3-(3-Fluoro-phenylethynyl)-3-hydroxy-cyclohexyl]-carbamic acid ethyl ester
(±)-(1RS,3SR)-[3-(3-Fluoro-phenylethynyl)-3-hydroxy-cyclohexyl]-carbamic acid ethyl ester
(±)-(1RS,3RS)-[3-(3-Methoxy-phenylethynyl)-3-hydroxy-cyclohexyl]-carbamic acid ethyl ester
(±)-(1RS,3RS)-N-[3-(3-Fluoro-phenylethynyl)-3-hydroxy-cyclohexyl]-acetamide.
(±)-(1RS,3SR)-N-[3-(3-Fluoro-phenylethynyl)-3-hydroxy-cyclohexyl]-acetamide
(±)-(1RS,3SR)-[3-Hydroxy-3-(3-methoxy-phenylethynyl)-cyclohexyl]-carbamic acid ethyl ester
(±)-(1RS,3RS)-N-[3-Hydroxy-3-(3-methoxy-phenylethynyl)-cyclohexyl]-acetamide
(±)-(1RS,3SR)-N-[3-Hydroxy-3-(3-methoxy-phenylethynyl)-cyclohexyl]-acetamide.
(±)-(1RS,3RS)-[3-Hydroxy-3-(3-methoxy-phenylethynyl)-cyclohexyl]-carbamic acid *tert*-butyl ester
(±)-(1RS,3SR)-[3-Hydroxy-3-(3-methoxy-phenylethynyl)-cyclohexyl]-carbamic acid *tert*-butyl ester
(±)-(1RS,3RS)-(3-Hydroxy-3-m-tolylethynyl-cyclohexyl)-carbamic acid tert-butyl ester
(±)-(1RS,3SR)-(3-Hydroxy-3-m-tolylethynyl-cyclohexyl)-carbamic acid tert-butyl ester
(±)-(1RS,3RS)-(3-(3-Fluoro-phenylethynyl)-3-hydroxy-cyclohexyl]-carbamic acid tert-butyl ester
(±)-(1RS,3SR)-(3-(3-Fluoro-phenylethynyl)-3-hydroxy-cyclohexyl]-carbamic acid tert-butyl ester
(±)-(1RS,3RS)-[3-(3-Fluoro-phenylethynyl)-3-hydroxy-cyclohexyl]-carbamic acid methyl ester
(±)-(1RS,3SR)-[3-(3-Fluoro-phenylethynyl)-3-hydroxy-cyclohexyl]-carbamic acid methyl ester
(±)-(3-Phenylethynyl-cyclohex-2-enyl)-carbamic acid ethyl ester and (±)-3-phenylethynyl-cyclohex-3-enyl)-carbamic acid ethyl ester
(±)-Methyl-(3-phenylethynyl-cyclohex-3-enyl)-carbamic acid ethyl ester
(±)-(4aRS,5RS,8aSR)-5-Hydroxy-5-phenylethynyl-octahydro-quinoline-1-carboxylic acid ethyl ester
(±)-[(4aRS,5SR,8aSR)-5-(3-Chloro-phenylethynyl)-5-hydroxy-octahydro-quinolin-1-yl]-furan-2-yl-methanone
(±)-[(4aRS,5RS,8aSR)-5-(3-Chloro-phenylethynyl)-5-hydroxy-octahydro-quinolin-1-yl]-furan-2-yl-methanone
(±)-(4aRS,5RS,8aSR)-5-(3-Chloro-phenylethynyl)-5-hydroxy-octahydro-quinoline-1-carboxylic acid tert-butyl ester
(±)-[(4aRS,5SR,8aSR)-5-(3-Chloro-phenylethynyl)-5-hydroxy-octahydro-quinolin-1-yl]-morpholin-4-yl-methanone
(±)-[(4aRS,5SR,8aSR)-5-(3-chloro-phenylethynyl)-5-hydroxy-octahydro-quinolin-1-yl]-(4-methyl-piperazin-1-yl)-methanone
(±)-(4aRS,5RS,8aSR)-5-(3-chloro-phenylethynyl)-5-hydroxy-octahydro-quinoline-1-carboxylic acid ethyl ester and (±)-(4aRS,5SR,8aSR)- 5-(3-chloro-phenylethynyl)-5-hydroxy-octahydro-quinoline-1-carboxylic acid ethyl ester
(±)-(4aRS,5SR,8aSR)- 5-Hydroxy-5-m-tolylethynyl-octahydro-quinoline-1-carboxylic acid ethyl ester
(±)-(4aRS,5RS,8aSR)- 5-Hydroxy-5-m-tolylethynyl-octahydro-quinoline-1-carboxylic acid ethyl ester.

In a further embodiment, the mGluR modulator is a compound of the formula (V), for exaple, as described in WO 2006/114262: 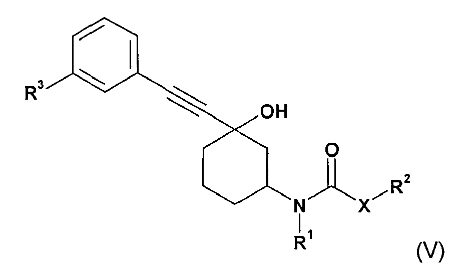 wherein
- R¹: represents hydrogen or alkyl;
- R²: represents an unsubstituted or substituted heterocycle or
- R²: represents an unsubstituted or substituted aryl;
- R³: represents alkyl or halogen;
- X: represents a single bond or an alkandiyl-group, optionally interrupted by one or more oxygen atoms or carbonyl groups or carbonyloxy groups
in free base or acid addition salt form.

Exemplary compounds of formula (V) include:
Furan-3-carboxylic acid [(1S,3S)-3-(3-chloro-phenylethynyl)-3-hydroxy-cyclohexyl]-amide
Furan-2-carboxylic acid [(1R,3R)-3-(3-chloro-phenylethynyl)-3-hydroxy-cyclohexyl]-amide
Furan-2-carboxylic acid [(1S,3S)-3-(3-chloro-phenylethynyl)-3-hydroxy-cyclohexyl]-amide
3H-imidazole-4-carboxylic acid [(1R,3R)-3-(3-chloro-phenylethynyl)-3-hydroxy-cyclohexyl]-amide
3H-Imidazole-4-carboxylic acid [(1S,3S)-3-(3-chloro-phenylethynyl)-3-hydroxy-cyclohexyl]-amide
4H-[1,2,4]Triazole-3-carboxylic acid [(1R,3R)-3-(3-chloro-phenylethynyl)-3-hydroxycyclohexyl]-amide
4H-[1,2,4]Triazole-3-carboxylic acid [(1S,3S)-3-(3-chloro-phenylethynyl)-3-hydroxycyclohexyl]-amide
2-Methyl-furan-3-carboxylic acid [(+)-(1R,3R)-3-(3-chforo-phenylethynyl)-3-hydroxycyclohexyl]-amide
N-[(±)-(1R,3R)-3-(3-Chloro-phenylethynyl)-3-hydroxy-cyclohexyl]-3,4-difluoro-benzamide
Benzo[1,3]dioxole-2-carboxylic acid [(±)-(1R,3R)-3-(3-chloro-phenylethynyl)-3-hydroxycyclohexyl]-amide
5-Methyl-pyrazine-2-carboxylic acid [(±)-(1R,3R)-3-(3-chloro-phenylethynyl)-3-hydroxycyclohexyl]-amide
Quinoxaline-2-carboxylic acid [(±)-(1R,3R)-3-(3-chloro-phenylethynyl)-3-hydroxy-cyclohexyl]-amide
Benzofuran-2-carboxylic acid [(±)- (1R,3R)-3-(3-chloro-phenylethynyl)-3-hydroxy-cyclohexyl]-amide
Benzooxazole-2-carboxylic acid [(±)- (1R,3R)-3-(3-chloro-phenylethynyl)-3-hydroxycyclohexyl]-amide
2,5-Dimethyl-furan-3-carboxylic acid [(±)- (1R,3R)-3-(3-chloro-phenylethynyl)-3-hydroxycyclohexyl]-amide
(R,S)-Tetrahydro-furan-3-carboxylic acid [(±)-(1R,3R)-3-(3-chloro-phenylethynyl)-3-hydroxycyclohexyl]-amide
Furan-3-carboxylic acid ((1R,3R)-3-hydroxy-3-m-tolylethynyl-cyclohexyl)-amide
Furan-3-carboxylic acid ((1S,3S)-3-hydroxy-3-m-tolylethynyl-cyclohexyl)-amide
Furan-3-carboxylic acid ((±)-(1R,3R)-3-hydroxy-3-m-tolylethynyl-cyclohexyl)-amide
Furan-2-carboxylic acid ((1R,3R)-3-hydroxy-3-m-tolylethynyl-cyclohexyl)-amide
Furan-2-carboxylic acid ((1S,3S)-3-hydroxy-3-m-tolylethynyl-cyclohexyl)-amide
Furan-2-carboxylic acid ((±)-(1R,3R)-3-hydroxy-3-m-tolylethynyl-cyclohexyl)-amide
Isoxazole-5-carboxylic acid ((1R,3R)-3-hydroxy-3-m-tolylethynyl-cyclohexyl)-amide
Isoxazole-5-carboxylic acid ((1S,3S)-3-hydroxy-3-m-tolylethynyl-cyclohexyl)-amide
Isoxazole-5-carboxylic acid ((±)-(1R,3R)-3-hydroxy-3-m-tolylethynyl-cyclohexyl)-amide
5-Methyl-pyrazine-2-carboxylic acid ((±)-(1R,3R)-3-hydroxy-3-m-tolylethynyl-cyclohexyl)-amide
4H-[1,2,4]Triazole-3-carboxylic acid ((±)-(1R,3R)-3-hydroxy-3-m-tolylethynyl-cyclohexyl)-amide
3H-imidazole-4-carboxylic acid ((±)- (1R,3R)-3-hydroxy-3-m-tolylethynyl-cyclohexyl)-amide Tetrahydro-pyran-4-carboxylic acid ((±)-(1R,3R)-3-hydroxy-3-m-tolylethynyl-cyclohexyl)-amide
1-Methyl-1H-imidazole-4-carboxylic acid ((±)-(1R,3R)-3-hydroxy-3-m-tolylethynyl-cyclohexyl)-amide
(R,S)-Tetrahydro-furan-2-carboxylic acid ((±)-(1R,3R)-3-hydroxy-3-m-tolylethynyl-cyclohexyl)-amide
(R,S)-Tetrahydro-furan-3-carboxylic acid ((±)-(1R,3R)-3-hydroxy-3-m-tolylethynyl-cyclohexyl)-amide
Furan-3-carboxylic acid [(1R,3R)-3-(3-fluoro-phenylethynyl)-3-hydroxy-cyclohexylj-amide
Furan-3-carboxylic acid [(1S,3S)-3-(3-fluoro-phenylethynyl)-3-hydroxy-cyclohexyl]-amide
Furan-2-carboxylic acid [(1R,3R)-3-(3-fluoro-phenylethynyl)-3-hydroxy-cyclohexyl]-amide
Furan-2-carboxylic acid [(1S,3S)-3-(3-fluoro-phenylethynyl)-3-hydroxy-cyclohexyl]-amide
3H-imidazole-4-carboxylic acid [(±)-(1R,3R)-3-(3-fluoro-phenylethynyl)-3-hydroxycyclohexyl]-amide
N-[(1S,3S)-3-(3-Chloro-phenylethynyl)-3-hydroxy-cyclohexylj-3,4-difluoro-benzamide
N-[(1R,3R)-3-(3-Chloro-phenylethynyl)-3-hydroxy-cyclohexyl]-3,4-difluoro-benzamide
Pyridine-2-carboxylic acid [(1S,3S)-3-(3-chloro-phenylethynyl)-3-hydroxy-cyclohexyl]-amide
Pyridine-2-carboxylic acid [(1R,3R)-3-(3-chloro-phenylethynyl)-3-hydroxy-cyclohexyl]-amide
N-[(1S,3S)-3-(3-Chloro-phenylethynyl)-3-hydroxy-cyclohexyl]-nicotinamide
N-[(1R,3R)-3-(3-Chloro-phenylethynyl)-3-hydroxy-cyclohexyl]-nicotinamide
Benzo[1,3]dioxole-2-carboxylic acid [(1S,3S)-3-(3-chloro-phenylethynyl)-3-hydroxycyclohexyl]-amide
5-Methyl-pyrazine-2-carboxylic acid [(1S,3S)-3-(3-chloro-phenylethynyl)-3-hydroxycyclohexyl]-amide
2-Methyl-furan-3-carboxylic acid [(1S,3S)-3-(3-chloro-phenylethynyl)-3-hydroxy-cyclohexyl]-amide
(R)-Tetrahydro-furan-2-carboxylic acid [(1R,3R)-3-(3-chloro-phenylethynyl)-3-hydroxycyclohexyl]-amide
(S)-Tetrahydro-furan-2-carboxylic acid [(1R,3R)-3-(3-chloro-phenylethynyl)-3-hydroxycyclohexyl]-amide
Isoxazole-5-carboxylic acid [(1S,3S)-3-(3-chloro-phenylethynyl)-3-hydroxy-cyclohexyl]-amide
5-Methyl-pyrazine-2-carboxylic acid [(1R,3R)-3-(3-chloro-phenylethynyl)-3-hydroxycyclohexyl]-amide
2-Methyl-furan-3-carboxylic acid [(1R,3R)-3-(3-chloro-phenylethynyl)-3-hydroxy-cyclohexyl]-amide
Isoxazole-5-carboxylic acid [(1R,3R)-3-(3-chloro-phenylethynyl)-3-hydroxy-cyclohexyl]-amide
5-Chloro-furan-2-carboxylic acid [(1R,3R)-3-(3-chloro-phenylethynyl)-3-hydroxy-cyclohexyl]-amide
5-Chloro-furan-2-carboxylic acid [(1S,3S)-3-(3-chloro-phenylethynyl)-3-hydroxy-cyclohexyl]-amide
(S)-Tetrahydro-furan-3-carboxylic acid [(1R,3R)-3-(3-chloro-phenylethynyl)-3-hydroxycyclohexyl]-amide
(R)-Tetrahydro-furan-3-carboxylic acid [(1R,3R)-3-(3-chloro-phenylethynyl)-3-hydroxycyclohexyl]-amide
N-[(1S,3S)-3-(3-Chloro-phenylethynyl)-3-hydroxy-cyclohexyl]-isonicotinamide
N-[(1R,3R)-3-(3-Chloro-phenylethynyl)-3-hydroxy-cyclohexyl]-isonicotinamide
3,5-Difluoro-pyridine-2-carboxylicacid [(1R,3R)-3-(3-chloro-phenylethynyl)-3-hydroxycyclohexyl]-amide
3,5-Difluoro-pyridine-2-carboxylicacid [(1S,3S)-3-(3-chloro-phenylethynyl)-3-hydroxycyclohexyl]-amide
6-Methyl-pyridine-2-carboxylic acid [(1S,3S)-3-(3-chloro-phenylethynyl)-3-hydroxycyclohexyl]-amide
6-Methyl-pyridine-2-carboxylic acid [(1R,3R)-3-(3-chloro-phenylethynyl)-3-hydroxycyclohexyl]-amide
5-Chloro-pyridine-2-carboxylic acid [(1R,3R)-3-(3-chloro-phenylethynyl)-3-hydroxycyclohexyl]-amide
5-Chloro-pyridine-2-carboxylic acid [(1S,3S)-3-(3-chloro-phenylethynyl)-3-hydroxycyclohexyl]-amide
6-Chloro-pyridine-2-carboxylic acid [(1R,3R)-3-(3-chloro-phenylethynyl)-3-hydroxycyclohexyl]-amide
6-Chloro-pyridine-2-carboxylic acid [(1S,3S)-3-(3-chlora-phenylethynyl)-3-hydroxycyclohexyl]-amide
5-Chloro-1-methyl-1H-pyrrole-2-carboxylic acid [(1R,3R)-3-(3-chloro-phenylethynyl)-3-hydroxy-cyclohexyl]-amide
5-Chloro-1-methyl-1H-pyrrole-2-carboxylic acid [(1S,3S)-3-(3-chloro-phenylethynyl)-3-hydroxy-cyclohexyl]-amide
5-Chloro-1H-pyrrole-2-carboxylic acid [(1R,3R)-3-(3-chloro-phenylethynyl)-3-hydroxycyclohexyl]-amide
5-Chloro-1H-pyrrole-2-carboxylic acid [(1S,3S)-3-(3-chloro-phenylethynyl)-3-hydroxycyclohexyl]-amide
N-[(1S,3S)-3-(3-Chloro-phenylethynyl)-3-hydroxy-cyclohexyl]-4-dimethyl amino-benzamide
1H-Pyrrole-3-carboxylic acid [(1S,3S)-3-(3-chloro-phenylethynyl)-3-hyd roxy-cyclohexyl]-amide
N-[(1S,3S)-3-(3-chloro-phenylethynyl)-3-hydroxy-cyclohexyl]-4-methyl-benzamide
N-[(1S,3S)-3-(3-Chloro-phenylethynyl)-3-hydroxy-cyclohexyl]-4-methyl-benzamide
N-[(1S,3S)-3-(3-Chloro-phenylethynyl)-3-hydroxy-cyclohexyl]-3-fluoro-benzamide
N-[(1S,3S)-3-(3-Chloro-phenylethynyl)-3-hydroxy-cyclohexyl]-2-ethyl-butyramide
N-[(1S,3S)-3-(3-Chloro-phenylethynyl)-3-hydroxy-cyclohexyl]-4-(2,5-dimethoxy-phenyl)-4-oxo-butyramide
2-(2-Benzyloxy-ethoxy)-N-[(1S,3S)-3-(3-chloro-phenylethynyl)-3-hydroxy-cyclohexyl]-acetamide
N-[(1S,3S)-3-(3-Chloro-phenylethynyl)-3-hydroxy-cyclohexyl)-2-phenyl-acetamide
N-[(1S,3S)-3-(3-Chloro-phenylethynyl)-3-hydroxy-cyclohexyl]-3-(1H-indol-4-yl)-propionamide
2-Benzo[1,3]dioxol-5-yl-N-[(1S,3S)-3-(3-chloro-phenylethynyl)-3-hydroxy-cyclohexyl]-acetamide
N-[(1S,3S)-3-(3-Chloro-phenylethynyl)-3-hydroxy-cyclohexyl]-2-phenoxy-propionamide
N-[(1S,3S)-3-(3-Chloro-phenylethynyl)-3-hydroxy-cyclohexyl]-2-(2-fluoro-phenyl)-acetamide
5-Hydroxy-1H-indole-2-carboxylic acid [(1S,3S)-3-(3-chloro-phenylethynyl)-3-hydroxycyclohexyl]-amide
1-Methyl-1H-pyrrole-2-carboxylic acid [(1S,3S)-3-(3-chloro-phenylethynyl)-3-hydroxycyclohexyl]-amide
N-[(1S,3S)-3-(3-Chloro-phenylethynyl)-3-hydroxy-cyclohexyl]-terephthalamic acid methyl ester
N-[(1S,3S)-3-(3-Chloro-phenylethynyl)-3-hydroxy-cyclohexyl]-2-(2-trifluoromethoxy-phenyl)-acetamide
5-Chloro-N-[(1S,3S)-3-(3-chloro-phenylethynyl)-3-hydroxy-cyclohexyl]-2-hydroxy-benzamide N-[(1S,3S)-3-(3-Chloro-phenylethynyl)-3-hydroxy-cyclohexyl]-4-hydroxy-benzamide
N-[(1S,3S)-3-(3-Chloro-phenylethynyl)-3-hydroxy-cyclohexyl]-2-hydroxy-benzamide
4-Amino-N-[(1S,3S)-3-(3-chloro-phenylethynyl)-3-hydroxy-cyclohexyl]-benzamide
4-Amino-5-chloro-N-[(1S,3S)-3-(3-chloro-phenylethynyl)-3-hydroxy-
3-Amino-4-chloro-N-[(1S,3S)-3-(3-chloro-phenylethynyl)-3-hydroxy-cyclohexyl]-benzamide
3-Amino-N-[(1S,3S)-3-(3-chloro-phenyfethynyl)-3-hydroxy-cyclohexyl]-4-methyl-benzamide
2-Amino-N-[(1S,3S)-3-(3-chloro-phenylethynyl)-3-hydroxy-cyclohexyl]-nicotinamide
N-[(1S,3S)-3-(3-Chloro-phenylethynyl)-3-hydroxy-cyclohexyl)-4-hydroxy-3-methoxybenzamide
N-[(1S,3S)-3-(3-Chloro-phenylethynyl)-3-hydroxy-cyclohexyl]-2-fluoro-benzamide
N-[(1S,3S)-3-(3-Chloro-phenylethynyl)-3-hydroxy-cyclohexyl]-4-methanesulfonyl-benzamide
Pyridine-2-carboxylic acid [(1S,3S)-3-(3-chloro-phenylethynyl)-3-hydroxy-cyclohexyl]-amide
3-Amino-pyrazine-2-carboxylic acid [(1S,3S)-3-(3-chloro-phenylethynyl)-3-hydroxycyclohexyl]-amide
6-Amino-N-[(1S,3S)-3-(3-chloro-phenylethynyl)-3-hydroxy-cyclohexyl]-nicotinamide
4-(4-Amino-benzoylamino)-benzoic acid [(1R,3R)-3-(3-chloro-phenylethynyl)-3-hydroxycyclohexyl]-amide
2,6-Dioxo-1,2,3,6-tetrahydro-pyrimidine-4-carboxylic acid [(1S,3S)-3-(3-chloro-phenylethynyl)-3-hydroxy-cyclohexyl]-amide
N-[(1S,3S)-3-(3-Chloro-phenylethynyl)-3-hydroxy-cyclohexyl]-isonicotinamide
3-Chloro-N-[(1S,3S)-3-(3-chloro-phenylethynyl)-3-hydroxy-cyclohexyl]-benzamide
N-[(1S,3S)-3-(3-Chloro-phenylethynyl)-3-hydroxy-cyclohexyl]-2,3-dimethoxy-benzamide
N-[(1S,3S)-3-(3-Chloro-phenylethynyl)-3-hydroxy-cyclohexyl]-4-oxo-4-phenyl-butyramide
2-Chloro-N-[(1S,3S)-3-(3-chloro-phenylethynyl)-3-hydroxy-cyclohexyl]-nicotinamide
5-Bromo-N-[(1S,3S)-3-(3-chloro-phenylethynyl)-3-hydroxy-cyclohexyl]-nicotinamide
Isoquinoline-1-carboxylic acid [(1S,3S)-3-(3-chloro-phenylethynyl)-3-hydroxy-cyclohexyl]-amide
Pyrazine-2-carboxylic acid [(1S,3S)-3-(3-chloro-phenylethynyl)-3-hydroxy-cyclohexyl]-amide
3-Benzoyl-pyridine-2-carboxylic acid [(1S,3S)-3-(3-chloro-phenylethynyl)-3-hydroxycyclohexyl]-amide
N-[(1S,3S)-3-(3-Chloro-phenylethynyl)-3-hydroxy-cyclohexyl]-2-methyl-nicotinamide
Quinoxaline-2-carboxylic acid [(1S,3S)-3-(3-chloro-phenylethynyl)-3-hydroxy-cyclohexyl]-amide
Pyridazine-4-carboxylic acid [(1S,3S)-3-(3-chloro-phenylethynyl)-3-hydroxy-cyclohexyl]-amide
N-[(1S,3S)-3-(3-Chloro-phenylethynyl)-3-hydroxy-cyclohexyl]-2-methylsulfanyl-nicotinamide
N-[(1S,3S)-3-(3-Chloro-phenylethynyl)-3-hydroxy-cyclohexyl]-4-trifluoromethyl-nicotinamide
2-Chloro-N-[(1S,3S)-3-(3-chloro-phenylethynyl)-3-hydroxy-cyclohexyl]-isonicotinamide
2-Chloro-N-[(1S,3S)-3-(3-chloro-phenylethynyl)-3-hydroxy-cyclohexyl]-6-methyl-nicotinamide
6-Chloro-N-[(1S,3S)-3-(3-chloro-phenylethynyl)-3-hydroxy-cyclohexyl]-nicotinamide
2-Chloro-N-[(1S,3S)-3-(3-chloro-phenylethynyl)-3-hydroxy-cyclohexyl]-6-methyl-isonicotinamide
N-[(1S,3S)-3-(3-Chloro-phenylethynyl)-3-hydroxy-cyclohexyl]-2-(4,5-dimethoxy-3-oxo-1,3-dihydro-isobenzofuran-1-yl)-acetamide
1,4,5,6-Tetrahydro-cyclopentapyrazole-3-carboxylic acid [(1S,3S)-3-(3-chloro-phenylethynyl)-3-hydroxy-cyclohexyl]-amide
N-[(1S,3S)-3-(3-Chloro-phenylethynyl)-3-hydroxy-cyclohexyl]-3-(1H-indol-2-yl)-propionamide
6-[(1S,3S)-3-(3-Chloro-phenylethynyl)-3-hydroxy-cyclohexylcarbamoyl]-pyridine-2-carboxylic acid isopropyl ester
Quinoline-6-carboxylic acid [(1S,3S)-3-(3-chloro-phenylethynyl)-3-hydroxy-cyclohexyl]-amide
5-Methyl-isoxazole-4-carboxylic acid [(1S,3S)-3-(3-chloro-phenylethynyl)-3-hydroxycyclohexyl]-amide
Benzofuran-3-carboxylic acid [(1S,3S)-3-(3-chloro-phenylethynyl)-3-hydroxy-cyclohexyl]-amide
N-[(1S,3S)-3-(3-Chloro-phenylethynyl)-3-hydroxy-cyclohexyl]-2-(2-methoxy-phenoxy)-acetamide.

In a further embodiment, the mGluR modulator is a compound of the formula (VI) 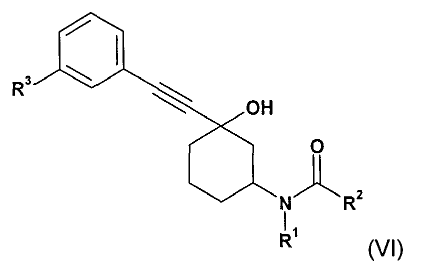 wherein
- R¹: represents hydrogen or alkyl;
- R²: represents an unsubstituted or substituted heterocycle or
- R²: represents an unsubstituted or substituted aryl;
- R³: represents alkyl or halogen;
in free base or acid addition salt form.

In another embodiment, the mGluR modulator is a compound of the formula (VII): 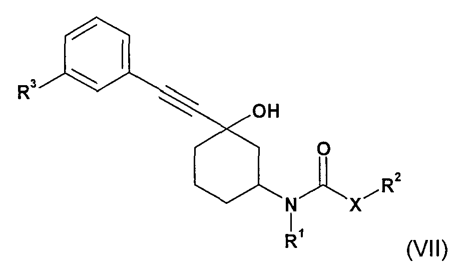 wherein
- R¹: represents hydrogen or alkyl;
- R²: represents an unsubstituted or substituted heterocycle or
- R²: represents an unsubstituted or substituted aryl;
- R³: represents alkyl or halogen;
- X: represents a single bond or an alkandiyl-group, optionally interrupted by one ore more oxygen atoms or carbonyl groups or carbonyloxy groups

in free base or acid addition salt form.

In a further embodiment, the invention provides a compound of formula (VIII) 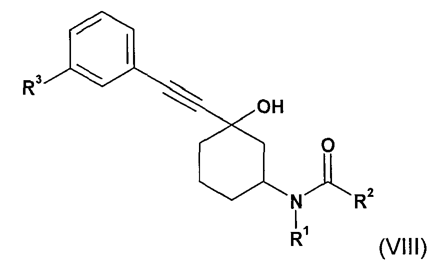 wherein
- R¹: represents hydrogen or alkyl;
- R²: represents an unsubstituted or substituted heterocycle or
- R²: represents an unsubstituted or substituted aryl;
- R³: represents alkyl or halogen;
in free base or acid addition salt form.

Preferred substituents, preferred ranges of numerical values or preferred ranges of the radicals present in the formula (VII) and formula (VIII) are defined below.
- R¹: preferably represents hydrogen or C₁₋₄ alkyl.
- R¹: particularly preferably represents hydrogen.
- R³: preferably represents Fluoro, Chloro, C₁₋₄ alkyl.
- R³: particularly preferably represents chloro or methyl.
- R²: preferably represents an unsubstituted or substituted heterocycle having 3 - 11 ring atoms and 1 - 4 hetero atoms; the hetero atoms being selected from the group consisting of N, O, S, the substituents being selected from the group consisting of Oxo (=O), Hydroxy, Halogen, Amino, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl.
- R²: further preferably represents phenyl or substituted phenyl, the substituents being selected from the group consisting of Hydroxy, Amino, Halogen, Nitro, Cyano, C₁₋₄ Alkyl, C₁-₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl.
- R²: particularly preferably represents an unsubstituted, a single or twofold substituted heterocycle having 5 - 9 ring atoms and 1 - 3 hetero atoms; the hetero atoms being selected from the group consisting of N, O; the substituents being selected from the group consisting of Halogen, C₁₋₄ Alkyl.
- R²: particularly preferably represents an unsubstituted, a single or twofold substituted phenyl, the substituents being selected from the group consisting of fluoro, chloro, hydroxy, methyl, methoxy, methoxycarbonyl, trifluormethoxy, amino, dimethylamino, methylthio, methylsulfonyl.
- R²: very particularly preferably represents an unsubstituted, a single or twofold substituted heterocycle selected from the group consisting of 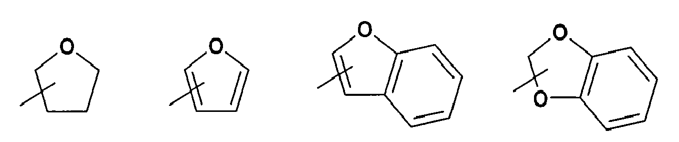 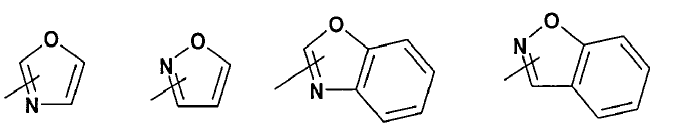 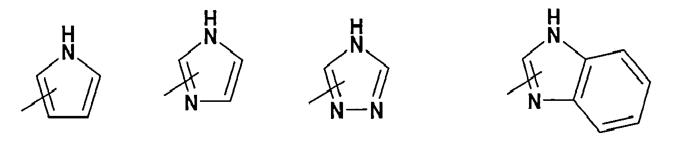 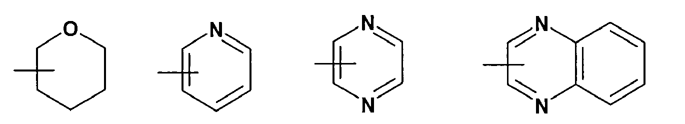 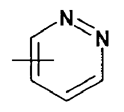 and the substituents selected from the group consisting of fluoro, chloro, methyl, methylthio, amino.
- R²: further very particularly preferably represents a substituent selected from the group consisting of 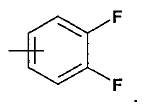
- X: preferably represents C₁₋₆ alkandiyl, C₁₋₆ alkandiyl with an oxygen group at the end or C₁₋₆ alkandiyl with an carbonyl group at the end, C₁₋₆ alkandiyl with an carbonyloxy group at the end.
- X: particular preferably represents, methandiyl (-CH₂-), 1,2-ethanediyl (-CH₂-CH₂-), 1,1- ethanediyl ((-CH(CH₃)-), , methandiyloxy (-O-CH₂-), 1,2-ethanediyloxy (-O-CH₂-CH₂-), 1,1-ethanediyloxy ((-O-CH(CH₃)-), methandiylcarbonyl (-CO-CH₂-), 1,2- ethanediylcarbonyl (-CO-CH₂-CH₂-), 1,1-ethanediylcarbonyl ((-CO-CH(CH₃)-), methandiylcarbonyloxy (-C(O)O-CH₂-), 1,2-ethanediylcarbonyloxy (-C(O)O-CH₂-CH₂-), 1,1-ethanediylcarbonyloxy ((-C(O)O-CH(CH₃)-). The functional groups as defined for X are preferably bound to the group R².

The abovementioned general or preferred radical definitions can be combined with one another at will, i.e. including combinations between the given preferred ranges. Further, individual definitions may not apply.

Preference according to the invention is given to compounds of the formula (VII) which contain a combination of the meanings mentioned above as being preferred.

Particular preference according to the invention is given to compounds of the formula (VII) which contain a combination of the meanings listed above as being particularly preferred.

Very particular preference according to the invention is given to the compounds of the formula (VII) which contain a combination of the meanings listed above as being very particularly preferred.

Preferred are compounds of formula (VII) wherein R² represents an unsubstituted or substituted heterocycle.

In a further embodiment, the invention provides a compound of formula (IX) 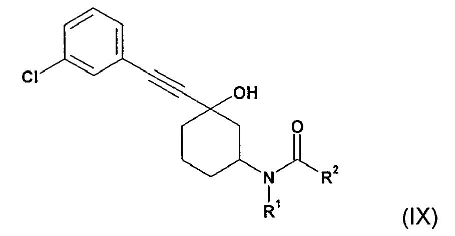 wherein R¹ and R² are as defined above.

In a further embodiment, the invention provides a compound of formula (IX) as defined above, wherein R² is as defined above and R¹ represents hydrogen.

Examples of compounds of formula (VII), (VIII) and (IX) include:
Furan-3-carboxylic acid [(1R,3R)-3-(3-chloro-phenylethynyl)-3-hydroxy-cyclohexyl]-amide
Furan-3-carboxylic acid [(1S,3S)-3-(3-chloro-phenylethynyl)-3-hydroxy-cyclohexyl]-amide
Furan-2-carboxylic acid [(1R,3R)-3-(3-chloro-phenylethynyl)-3-hydroxy-cyclohexyl]-amide
Furan-2-carboxylic acid [(1S,3S)-3-(3-chloro-phenylethynyl)-3-hydroxy-cyclohexyl]-amide
3H-Imidazole-4-carboxylic acid [(1R,3R)-3-(3-chloro-phenylethynyl)-3-hydroxy-cyclohexyl]-amide
3H-Imidazole-4-carboxylic acid [(1S,3S)-3-(3-chloro-phenylethynyl)-3-hydroxy-cyclohexyl]-amide
4H-[1,2,4]Triazole-3-carboxylic acid [(1R,3R)-3-(3-chloro-phenylethynyl)-3-hydroxycyclohexyl]-amide
4H-[1,2,4]Triazole-3-carboxylic acid [(1S,3S)-3-(3-chtoro-pheny)ethynyl)-3-hydroxycyclohexyl]-amide
2-Methyl-furan-3-carboxylic acid [(±)-(1R,3R)-3-(3-chloro-phenylethynyl)-3-hydroxycyclohexyl]-amide
N-[(±)-(1R,3R)-3-(3-Chloro-phenylethynyl)-3-hydroxy-cyclohexyl]-3,4-difluoro-benzamide
Benzo[1,3]dioxole-2-carboxylic acid [(±)-(1R,3R)-3-(3-chloro-phenylethynyl)-3-hydroxycyclohexyl]-amide
5-Methyl-pyrazine-2-carboxylic acid [(±)-(1R,3R)-3-(3-chloro-phenylethynyl)-3-hydroxycyclohexyl]-amide
Quinoxaline-2-carboxylic acid [(±)-(1R,3R)-3-(3-chloro-phenylethynyl)-3-hydroxy-cyclohexyl]-amide
Benzofuran-2-carboxylic acid [(±)- (1R,3R)-3-(3-chloro-phenylethynyl)-3-hydroxy-cyclohexyl]-amide
Benzooxazole-2-carboxylic acid [(±)- (1R,3R)-3-(3-chloro-phenylethynyl)-3-hydroxycyclohexyl]-amide
2,5-Dimethyl-furan-3-carboxylic acid [(±)- (1R,3R)-3-(3-chloro-phenylethynyl)-3-hydroxycyclohexyl]-amide
(R,S)-Tetrahydro-furan-3-carboxylic acid [(±)-(1R,3R)-3-(3-chloro-phenylethynyl)-3-hydroxycyclohexyl]-amide
Furan-3-carboxylic acid ((1R,3R)-3-hydroxy-3-m-tolylethynyl-cyclohexyl)-amide
Furan-3-carboxylic acid ((1S,3S)-3-hydroxy-3-m-tolylethynyl-cyclohexyl)-amide
Furan-3-carboxylic acid ((±)-(1R,3R)-3-hydroxy-3-m-tolylethynyl-cyclohexyl)-amide
Furan-2-carboxylic acid ((1R,3R)-3-hydroxy-3-m-tolylethynyl-cyclohexyl)-amide
Furan-2-carboxylic acid ((1S,3S)-3-hydroxy-3-m-tolylethynyl-cyclohexyl)-amide
Furan-2-carboxylic acid ((±)-(1R,3R)-3-hydroxy-3-m-tolylethynyl-cyclohexyl)-amide
lsoxazole-5-carboxylic acid ((1R,3R)-3-hydroxy-3-m-tolylethynyl-cyclohexyl)-amide
lsoxazole-5-carboxylic acid ((1S,3S)-3-hydroxy-3-m-tolylethynyl-cyclohexyl)-amide
Isoxazole-5-carboxylic acid ((±)-(1R,3R)-3-hydroxy-3-m-tolylethynyl-cyclohexyl)-amide
5-Methyl-pyrazine-2-carboxylic acid ((±)-(1R,3R)-3-hydroxy-3-m-tolylethynyl-cyclohexyl)-amide
4H-[1,2,4]Triazole-3-carboxylic acid ((±)-(1R,3R)-3-hydroxy-3-m-tolylethynyl-cyclohexyl)-amide
3H-Imidazole-4-carboxylic acid ((±)- (1R,3R)-3-hydroxy-3-m-tolylethynyl-cyclohexyl)-amide
Tetrahydro-pyran-4-carboxylic acid ((±)-(1R,3R)-3-hydroxy-3-m-tolylethynyl-cyclohexyl)-amide
1-Methyl-1H-imidazole-4-carboxylic acid ((±)-(1R,3R)-3-hydroxy-3-m-tolylethynyl-cyclohexyl)-amide
(R,S)-Tetrahydro-furan-2-carboxylic acid ((±)-(1R,3R)-3-hydroxy-3-m-tolylethynylcyclohexyl)-amide
(R,S)-Tetrahydro-furan-3-carboxylic acid ((±)-(1R,3R)-3-hydroxy-3-m-tolylethynylcyclohexyl)-amide
Furan-3-carboxylic acid [(1R,3R)-3-(3-fluoro-phenylethynyl)-3-hydroxy-cyclohexyl]-amide
Furan-3-carboxylic acid [(1S,3S)-3-(3-fluoro-phenylethynyl)-3-hydroxy-cyclohexyl]-amide
Furan-2-carboxylic acid [(1R,3R)-3-(3-fluoro-phenylethynyl)-3-hydroxy-cyclohexyl]-amide
Furan-2-carboxylic acid [(1S,3S)-3-(3-fluoro-phenylethynyl)-3-hydroxy-cyclohexyl]-amide
3H-Imidazole-4-carboxylic acid [(±)-(1R,3R)-3-(3-fluoro-phenylethynyl)-3-hydroxycyclohexyl]-amide
N-[(1S,3S)-3-(3-Chloro-phenylethynyl)-3-hydroxy-cyclohexyl]-3,4-difluoro-benzamide
N-[(1R,3R)-3-(3-Chloro-phenylethynyl)-3-hydroxy-cyclohexyl]-3,4-difluoro-benzamide
Pyridine-2-carboxylic acid [(1S,3S)-3-(3-chloro-phenylethynyl)-3-hydroxy-cyclohexyl]-amide
Pyridine-2-carboxylic acid [(1R,3R)-3-(3-chloro-phenylethynyl)-3-hydroxy-cyclohexyl]-amide
N-[(1S,3S)-3-(3-Chloro-phenylethynyl)-3-hydroxy-cyclohexyl]-nicotinamide
N-[(1R,3R)-3-(3-Chloro-phenylethynyl)-3-hydroxy-cyclohexyl]-nicotinamide
Benzo[1,3]dioxole-2-carboxylic acid [(1S,3S)-3-(3-chloro-phenylethynyl)-3-hydroxycyclohexyl]-amide
5-Methyl-pyrazine-2-carboxylic acid [(1S,3S)-3-(3-chloro-phenylethynyl)-3-hydroxycyclohexyl]-amide
2-Methyl-furan-3-carboxylic acid [(1S,3S)-3-(3-chloro-phenylethynyl)-3-hydroxy-cyclohexyl]-amide
(R)-Tetrahydro-furan-2-carboxylic acid [(1R,3R)-3-(3-chloro-phenylethynyl)-3-hydroxycyclohexyl]-amide
(S)-Tetrahydro-furan-2-carboxylic acid [(1R,3R)-3-(3-chloro-phenylethynyl)-3-hydroxycyclohexyl]-amide
lsoxazole-5-carboxylic acid [(1S,3S)-3-(3-chloro-phenylethynyl)-3-hydroxy-cyclohexyl]-amide
5-Methyl-pyrazine-2-carboxylic acid [(1R,3R)-3-(3-chloro-phenylethynyl)-3-hydroxycyclohexyl]-amide
2-Methyl-furan-3-carboxylic acid [(1R,3R)-3-(3-chloro-phenylethynyl)-3-hydroxy-cyclohexyl]-amide
lsoxazole-5-carboxylic acid [(1R,3R)-3-(3-chloro-phenylethynyl)-3-hydroxy-cyclohexyl]-amide
5-Chloro-furan-2-carboxylic acid [(1R,3R)-3-(3-chloro-phenylethynyl)-3-hydroxy-cyclohexyl]-amide
5-Chloro-furan-2-carboxylic acid [(1S,3S)-3-(3-chloro-phenylethynyl)-3-hydroxy-cyclohexyl]-amide
(S)-Tetrahydro-furan-3-carboxylic acid [(1R,3R)-3-(3-chloro-phenylethynyl)-3-hydroxycyclohexyl]-amide
(R)-Tetrahydro-furan-3-carboxylic acid [(1R,3R)-3-(3-chloro-phenylethynyl)-3-hydroxycyclohexyl]-amide
N-[(1S,3S)-3-(3-Chloro-phenylethynyl)-3-hydroxy-cyclohexyl]-isonicotinamide
N-[(1R,3R)-3-(3-Chloro-phenylethynyl)-3-hydroxy-cyclohexyl]-isonicotinamide
3,5-Difluoro-pyridine-2-carboxylicacid [(1R,3R)-3-(3-chloro-phenylethynyl)-3-hydroxycyclohexyl]-amide
3,5-Difluoro-pyridine-2-carboxylicacid [(1S,3S)-3-(3-chloro-phenylethynyl)-3-hydroxycyclohexyl]-amide
6-Methyl-pyridine-2-carboxylic acid [(1S,3S)-3-(3-chloro-phenylethynyl)-3-hydroxycyclohexyl]-amide
6-Methyl-pyridine-2-carboxylic acid [(1R,3R)-3-(3-chloro-phenylethynyl)-3-hydroxycyclohexyl]-amide
5-Chloro-pyridine-2-carboxylic acid [(1R,3R)-3-(3-chloro-phenylethynyl)-3-hydroxycyclohexyl]-amide
5-Chloro-pyridine-2-carboxylic acid [(1S,3S)-3-(3-chloro-phenylethynyl)-3-hydroxycyclohexyl]-amide
6-Chloro-pyridine-2-carboxylic acid [(1R,3R)-3-(3-chloro-phenylethynyl)-3-hydroxycyclohexyl]-amide
6-Chloro-pyridine-2-carboxylic acid [(1S,3S)-3-(3-chloro-phenylethynyl)-3-hydroxycyclohexyl]-amide
5-Chloro-1-methyl-1H-pyrrole-2-carboxylic acid [(1R,3R)-3-(3-chloro-phenylethynyl)-3-hydroxy-cyclohexyl]-amide
5-Chloro-1-methyl-1H-pyrrole-2-carboxylic acid [(1S,3S)-3-(3-chloro-phenylethynyl)-3-hydroxy-cyclohexyl]-amide
5-Chloro-1H-pyrrole-2-carboxylic acid [(1R,3R)-3-(3-chloro-phenylethynyl)-3-hydroxycyclohexyl]-amide
5-Chloro-1H-pyrrole-2-carboxylic acid [(1S,3S)-3-(3-chloro-phenylethynyl)-3-hydroxycyclohexyl]-amide
N-[(1S,3S)-3-(3-Chloro-phenylethynyl)-3-hydroxy-cyclohexyl]-4-dimethyl amino-benzamide
1H-Pyrrole-3-carboxylic acid [(1S,3S)-3-(3-chloro-phenylethynyl)-3-hyd roxy-cyclohexyl]-amide
N-[(1S,3S)-3-(3-chloro-phenylethynyl)-3-hydroxy-cyclohexyl]-4-methyl-benzamide
N-[(1S,3S)-3-(3-Chloro-phenylethynyl)-3-hydroxy-cyclohexyl]-4-methyl-benzamide
N-[(1S,3S)-3-(3-Chloro-phenylethynyl)-3-hydroxy-cyclohexyl]-3-fluoro-benzamide
N-[(1S,3S)-3-(3-Chloro-phenylethynyl)-3-hydroxy-cyclohexyl]-2-ethyl-butyramide
N-[(1S,3S)-3-(3-Chloro-phenylethynyl)-3-hydroxy-cyclohexyl]-4-(2,5-dimethoxy-phenyl)-4-oxo-butyramide
2-(2-Benzyloxy-ethoxy)-N-[(1S,3S)-3-(3-chloro-phenylethynyl)-3-hydroxy-cyclohexyl]-acetamide
N-[(1S,3S)-3-(3-Chloro-phenylethynyl)-3-hydroxy-cyclohexyl]-2-phenyl-acetamide
N-[(1S,3S)-3-(3-Chloro-phenylethynyl)-3-hydroxy-cyclohexyl]-3-(1H-indol-4-yl)-propionamide
2-Benzo[1,3]dioxol-5-yl-N-[(1S,3S)-3-(3-chloro-phenylethynyl)-3-hydroxy-cyclohexyl]-acetamide
N-[(1S,3S)-3-(3-Chloro-phenylethynyl)-3-hydroxy-cyclohexyl]-2-phenoxy-propionamide
N-[(1S,3S)-3-(3-Chloro-phenylethynyl)-3-hydroxy-cyclohexyl]-2-(2-fluoro-phenyl)-acetamide
5-Hydroxy-1H-indole-2-carboxylic acid [(1S,3S)-3-(3-chloro-phenylethynyl)-3-hydroxycyclohexyl]-amide
1-Methyl-1H-pyrrole-2-carboxylic acid [(1S,3S)-3-(3-chloro-phenylethynyl)-3-hydroxycyclohexyl]-amide
N-[(1S,3S)-3-(3-Chloro-phenylethynyl)-3-hydroxy-cyclohexyl]-terephthalamic acid methyl ester
N-[(1S,3S)-3-(3-Chloro-phenylethynyl)-3-hydroxy-cyclohexyl]-2-(2-trifluoromethoxy-phenyl)-acetamide
5-Chloro-N-[(1S,3S)-3-(3-chloro-phenylethynyl)-3-hydroxy-cyclohexyl]-2-hydroxy-benzamide N-[(1S,3S)-3-(3-Chloro-phenylethynyl)-3-hydroxy-cyclohexyl]-4-hydroxy-benzamide
N-[(1S,3S)-3-(3-Chloro-phenylethynyl)-3-hydroxy-cyclohexyl]-2-hydroxy-benzamide
4-Amino-N-[(1S,3S)-3-(3-chloro-phenylethynyl)-3-hydroxy-cyclohexyl]-benzamide
4-Amino-5-chloro-N-[(1S,3S)-3-(3-chloro-phenylethynyl)-3-hydroxy-cyclohexyl]-2-methoxybenzamide
3-Amino-4-chloro-N-[(1S,3S)-3-(3-chloro-phenylethynyl)-3-hydroxy-cyclohexyl]-benzamide
3-Amino-N-[(1S,3S)-3-(3-chloro-phenylethynyl)-3-hydroxy-cyclohexyl]-4-methyl-benzamide
2-Amino-N-[(1S,3S)-3-(3-chloro-phenylethynyl)-3-hydroxy-cyclohexyl]-nicotinamide
N-[(1S,3S)-3-(3-Chloro-phenylethynyl)-3-hydroxy-cyclohexyl]-4-hydroxy-3-methoxybenzamide
N-[(1S,3S)-3-(3-Chloro-phenylethynyl)-3-hydroxy-cyclohexyl]-2-fluoro-benzamide
N-[(1S,3S)-3-(3-Chloro-phenylethynyl)-3-hydroxy-cyclohexyl]-4-methanesulfonyl-benzamide
Pyridine-2-carboxylic acid [(1S,3S)-3-(3-chloro-phenylethynyl)-3-hydroxy-cyclohexyl]-amide
3-Amino-pyrazine-2-carboxylic acid [(1S,3S)-3-(3-chloro-phenylethynyl)-3-hydroxycyclohexyl]-amide
6-Amino-N-[(1S,3S)-3-(3-chloro-phenylethynyl)-3-hydroxy-cyclohexyl]-nicotinamide
4-(4-Amino-benzoylamino)-benzoic acid [(1R,3R)-3-(3-chloro-phenylethynyl)-3-hydroxycyclohexyl]-amide
2,6-Dioxo-1,2,3,6-tetrahydro-pyrimidine-4-carboxylic acid [(1S,3S)-3-(3-chlorophenylethynyl)-3-hydroxy-cyclohexyl]-amide
N-[(1S,3S)-3-(3-Chloro-phenylethynyl)-3-hydroxy-cyclohexyl]-isonicotinamide
3-Chloro-N-[(1S,3S)-3-(3-chloro-phenylethynyl)-3-hydroxy-cyclohexyl]-benzamide
N-[(1S,3S)-3-(3-Chloro-phenylethynyl)-3-hydroxy-cyclohexyl]-2,3-dimethoxy-benzamide
N-[(1S,3S)-3-(3-Chloro-phenylethynyl)-3-hydroxy-cyclohexyl]-4-oxo-4-phenyl-butyramide
2-Chloro-N-[(1S,3S)-3-(3-chloro-phenylethynyl)-3-hydroxy-cyclohexyl]-nicotinamide
5-Bromo-N-[(1S,3S)-3-(3-chloro-phenylethynyl)-3-hydroxy-cyclohexyl]-nicotinamide
Isoquinoline-1-carboxylic acid [(1S,3S)-3-(3-chloro-phenylethynyl)-3-hydroxy-cyclohexyl]-amide
Pyrazine-2-carboxylic acid [(1S,3S)-3-(3-chloro-phenylethynyl)-3-hydroxy-cyclohexyl]-amide
3-Benzoyl-pyridine-2-carboxylic acid [(1S,3S)-3-(3-chloro-phenylethynyl)-3-hydroxycyclohexyl]-amide
N-[(1S,3S)-3-(3-Chloro-phenylethynyl)-3-hydroxy-cyclohexyl]-2-methyl-nicotinamide
Quinoxaline-2-carboxylic acid [(1S,3S)-3-(3-chloro-phenylethynyl)-3-hydroxy-cyclohexyl]-amide
Pyridazine-4-carboxylic acid [(1S,3S)-3-(3-chloro-phenylethynyl)-3-hydroxy-cyclohexyl]-amide
N-[(1S,3S)-3-(3-Chloro-phenylethynyl)-3-hydroxy-cyclohexyl]-2-methylsulfanyl-nicotinamide
N-[(1S,3S)-3-(3-Chloro-phenylethynyl)-3-hydroxy-cyclohexyl]-4-trifluoromethyl-nicotinamide
2-Chloro-N-[(1S,3S)-3-(3-chloro-phenylethynyl)-3-hydroxy-cyclohexyl]-isonicotinamide
2-Chloro-N-[(1S,3S)-3-(3-chloro-phenylethynyl)-3-hydroxy-cyclohexyl]-6-methyl-nicotinamide
6-Chloro-N-[(1S,3S)-3-(3-chloro-phenylethynyl)-3-hydroxy-cyclohexyl]-nicotinamide
2-Chloro-N-[(1S,3S)-3-(3-chloro-phenylethynyl)-3-hydroxy-cyclohexyl]-6-methyl-isonicotinamide
N-[(1S,3S)-3-(3-Chloro-phenylethynyl)-3-hydroxy-cyclohexyl]-2-(4,5-dimethoxy-3-oxo-1,3-dihydro-isobenzofuran-1-yl)-acetamide
1,4,5,6-Tetrahydro-cyclopentapyrazole-3-carboxylic acid [(1S,3S)-3-(3-chloro-phenylethynyl)-3-hydroxy-cyclohexyl]-amide
N-[(1S,3S)-3-(3-Chloro-phenylethynyl)-3-hydroxy-cyclohexyl]-3-(1H-indol-2-yl)-propionamide
6-[(1S,3S)-3-(3-Chloro-phenylethynyl)-3-hydroxy-cyclohexylcarbamoyl]-pyridine-2-carboxylic acid isopropyl ester
Quinoline-6-carboxylic acid [(1S,3S)-3-(3-chloro-phenylethynyl)-3-hydroxy-cyclohexyl]-amide
5-Methyl-isoxazole-4-carboxylic acid [(1S,3S)-3-(3-chloro-phenylethynyl)-3-hydroxycyclohexyl]-amide
Benzofuran-3-carboxylic acid [(1S,3S)-3-(3-chloro-phenylethynyl)-3-hydroxy-cyclohexyl]-amide
N-[(1S,3S)-3-(3-Chloro-phenylethynyl)-3-hydroxy-cyclohexyl]-2-(2-methoxy-phenoxy)-acetamide.

Further examples of mGluR, in particular mGluR5, modulators include compounds of the formula (I) as defined in WO 2004/014881 and compounds of the formula (I) as defined in WO 2007/021575; the contents of these publications are incorporated herein by reference.

Compounds of the invention and their pharmaceutically acceptable acid addition salts, hereinafter referred to as agents of the invention, exhibit valuable pharmacological properties and are therefore useful as pharmaceuticals.

Compounds of the invention may exhibit a marked and selective modulating, especially antagonistic, action at human mGluRs, in particular mGluR5s. This can be determined in vitro for example at recombinant human metabotropic glutamate receptors, especially PLC-coupled subtypes thereof such as mGluR5, using different procedures like, for example, measurement of the inhibition of the agonist induced elevation of intracellular Ca²⁺ concentration in accordance with L. P. Daggett et al., Neuropharm. Vol. 34, pages 871-886 (1995), P. J. Flor et al., J. Neurochem. Vol. 67, pages 58-63 (1996) or by determination to what extent the agonist induced elevation of the inositol phosphate turnover is inhibited as described by T. Knoepfel et al., Eur. J. Pharmacol. Vol. 288, pages 389-392 (1994), L. P. Daggett et al., Neuropharm. Vol. 67, pages 58-63 (1996) and references cited therein. Isolation and expression of human mGluR subtypes are described in US-Patent No. 5,521,297. Selected agents of the invention show IC50 values for the inhibition of the agonist (e.g. glutamate or quisqualate) induced elevation of intracellular Ca2+ concentration or the agonist (e.g. glutamate or quisqualate) induced inositol phosphate turnover, measured in recombinant cells expressing hmGluR5a of about 1 nM to about 50 µM.

Compounds of the invention are useful in the treatment, prevention or delay of progression of cognitive dysfunction. Cognitive dysfunction include deficits and abnormalities in attention and vigilance, executive functions and memory (for instance working memory and episodic memory). Other disorders relating to cognitive dysfunction include sleep related breathing disorders (SRBD), behavioural impairments, information processing deficits and age-related disorders.

Further examples of cognitive impairment and related disorders include attention-deficit hyperactivity disorder (ADHD), childhood ADHD, adult ADHD, excess daytime somnolence, sleep apnea, shift-worker's sleep-wake cycle disruption, traumatic brain injury, neurodegenerative disorders with associated memory and cognitive problems (such as Alzheimer's disease, Lewy body dementia, senile dementia, vascular dementia, Parkinson's disease), chronic fatigue syndrome, fatigue associated with sleep deprivation or prolonged wakefulness, age-related decline in memory and cognitive function (such as mild cognitive impairment), cognitive impairment associated with mood disorders (such as depression) and anxiety, schizophrenia and day time sleepiness associated with narcolepsy.

Treatment may comprise cognitive enhancement. The term "cognitive enhancement" includes, but is not limited to, cognition enhancement, vigilance, counteracting effects of fatigue, enhancing alertness, attention, memory (working, episodic), learning ability, reaction time, cognitive performance enhancement, excess daytime somnolence, reversal of information processing deficits, improvement of disorganization, i.e. improving organizational skills/level of organizational ability.

For the above-mentioned indications (the conditions and disorders) the appropriate dosage will vary depending upon, for example, the compound employed, the host, the mode of administration and the nature and severity of the condition being treated. However, in general, satisfactory results in animals are indicated to be obtained at a daily dosage of from about 0.01 to about 100 mg/kg body weight, preferably from about 0.1 to about 10 mg/kg body weight, e.g. 1 mg/kg. In larger mammals, for example humans, an indicated daily dosage is in the range from about 0.1 to about 1000 mg, preferably from about 1 to about 400 mg, most preferably from about 10 to about 100 mg of an mGluR, e.g. mGluR5, antagonist or other modulator conveniently administered, for example, in divided doses up to four times a day.

For use according to the invention, an mGluR modulator (e.g. an mGluR5 modulator, in particular an mGluR5 antagonist) may be administered as single active agent or in combination with other active agents, in any usual manner, e.g. orally, for example in the form of tablets or capsules, or parenterally, for example in the form of injection solutions or suspensions.

Moreover, the present invention provides a pharmaceutical composition comprising an mGluR modulator (e.g. an mGluR5 modulator, in particular an mGluR5 antagonist) in association with at least one pharmaceutical carrier or diluent for use in the treatment of cognitive dysfunction. Such compositions may be manufactured in conventional manner. Unit dosage forms may contain, for example, from about 2.5 to about 25 mg of one or more mGluR modulator, e.g. mGluR5 antagonist or other modulator.

The usefulness of the compounds of the invention in the treatment of the above-mentioned disorders can be confirmed in a range of standard tests including those indicated below:
The pharmaceutical compositions according to the invention are compositions for enteral, such as nasal, rectal or oral, or parenteral, such as intramuscular or intravenous, administration to warm-blooded animals (human beings and animals) that comprise an effective dose of the pharmacological active ingredient alone or together with a significant amount of a pharmaceutically acceptable carrier. The dose of the active ingredient depends on the species of warm-blooded animal, body weight, age and individual condition, individual pharmacokinetic data, the disease to be treated and the mode of administration.

The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, dragées, tablets or capsules.

The pharmaceutical compositions of the present invention are prepared in a manner known per se, for example by means of conventional dissolving, lyophilizing, mixing, granulating or confectioning processes.

The following numbered paragraphs are of interest to or illustrative of the invention.
1. The use of an mGluR modulator for the treatment, prevention or delay of progression of cognitive dysfunction.
2. Use according to numbered paragraph 1, wherein the modulator is an mGluR5 modulator.
3. Use according to numbered paragraph 1 or numbered paragraph 2, wherein the modulator is an mGluR antagonist.
4. Use according to numbered paragraph 2 or numbered paragraph 3, wherein the modulator is an mGluR5 antagonist.
5. Use according to any preceding numbered paragraph, wherein the modulator is a compound of the formula (I) 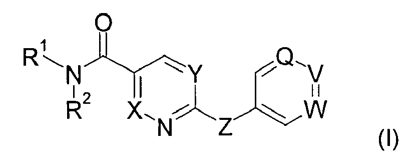 wherein
   - R¹: represents optionally substituted alkyl or optionally substituted benzyl; and
   - R²: represents hydrogen (H), optionally substituted alkyl or optionally substituted benzyl; or
   - R¹ and R²: form together with the nitrogen atom to which they are attached an optionally substituted heterocycle with less than 14 ring atoms;
   - R³: represents halogen, alkyl, alkoxy, alkylamino or dialkylamino;
   - R⁴: represents hydroxy (OH), halogen, alkyl or alkoxy;
   - Q: represents CH, CR⁴ or N;
   - V: represents CH, CR⁴ or N;
   - W: represents CH, CR⁴ or N;
   - X: represents CH or N;
   - Y: represents CH, CR³ or N;
   - Z: represents CH₂, NH or O; and
   provided that Q, V and W are not N at the same time;
   in free base or acid addition salt form.
6. Use according to any of numbered paragraphs 1 to 4, wherein the modulator is a compound of the formula (II), wherein a compound of the formula (II) is a compound of formula (I) in which at least one of Q, V and W is N, in free base or acid addition salt form.
7. Use according to any of numbered paragraphs 1 to 4, wherein the modulator is a compound of the formula (IV) or the formula (V): 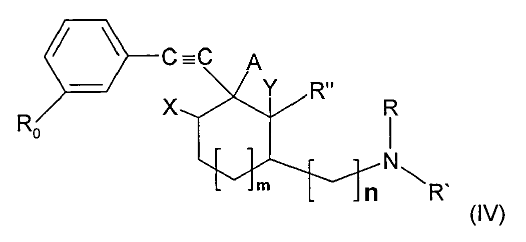 wherein
   m is 0 or 1,
   n is 0 or 1 and
   A is hydroxy
   X is hydrogen and
   Y is hydrogen, or
   A forms a single bond with X or with Y;
   R₀ is hydrogen, (C₁₋₄)alkyl, (C₁₋₄)alkoxy, trifluoromethyl, halogen, cyano, nitro, -COOR₁ wherein R₁ is (C₁₋₄)alkyl or -COR₂ wherein R₂ is hydrogen or (C₁₋₄)alkyl, and
   R is -COR₃, -COOR₃, -CONR₄R₅ or -SO₂R₆, wherein R₃ is (C₁₋₄)alkyl, (C₃₋₇)cycloalkyl or optionally substituted phenyl, 2-pyridyl or 2-thienyl; R₄ and R₅, independently, are hydrogen or (C₁₋₄)alkyl; and R₆ is (C₁₋₄)alkyl, (C₃₋₇)cycloalkyl or optionally substituted phenyl,
   R' is hydrogen or (C₁₋₄)alkyl and
   R" is hydrogen or (C₁₋₄)alkyl, or
   R' and R" together form a group -CH₂-(CH₂)ₘ-
   wherein m is 0, 1 or 2, in which case one of n and m is different from 0,
   with the proviso that R₀ is different from hydrogen, trifluoromethyl and methoxy when n is 0, A is hydroxy, X and Y are both hydrogen, R is COOEt and R' and R" together form a group - (CH₂)₂-,
   OR 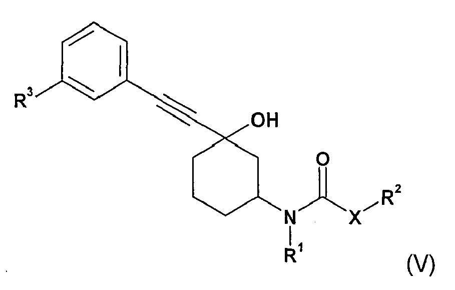 wherein
      - R¹: represents hydrogen or alkyl;
      - R²: represents an unsubstituted or substituted heterocycle or
      - R²: represents an unsubstituted or substituted aryl;
      - R³: represents alkyl or halogen;
      - X: represents a single bond or an alkandiyl-group, optionally interrupted by one or more oxygen atoms or carbonyl groups or carbonyloxy groups;
      in free base or acid addition salt form.
8. Use according to any preceding numbered paragraph, wherein the cognitive dysfunction comprises a disorder selected from deficits and abnormalities in attention and vigilance, executive functions and memory (for instance working memory and episodic memory), sleep related breathing disorders (SRBD), behavioral impairments, information processing deficits and age-related disorders.
9. Use according to any preceding numbered paragraph, wherein the cognitive dysfunction is associated with a disorder selected from sleep related breathing disorders (SRBDs), behavioral impairments, attention-deficit hyperactivity disorder (ADHD), childhood ADHD, adult ADHD, excess daytime somnolence, sleep apnea, shift-worker's sleep-wake cycle disruption, traumatic brain injury, neurodegenerative disorders with associated memory, cognitive problems (such as Alzheimer's disease, Lewy body dementia, senile dementia, vascular dementia, Parkinson's disease), chronic fatigue syndrome, fatigue associated with sleep deprivation or prolonged wakefulness, age-related decline in memory and cognitive function (such as mild cognitive impairment), mood disorders (such as depression), anxiety, schizophrenia and daytime sleepiness associated with narcolepsy.
10. A method for the treatment, prevention or delay of progression of cognitive dysfunction in a subject in need of such treatment, which comprises administering to said subject a therapeutically effective amount of an mGluR modulator.
11. A method according to numbered paragraph 10, wherein the mGluR modulator is as defined in any of numbered paragraphs 2 to 7.
12. A method according to numbered paragraph 10 or numbered paragraph 11, wherein the mGluR modulator is an mGluR5 modulator.
13. A method according to numbered paragraph 12, wherein the mGluR modulator is an mGluR5 antagonist.
14. A method according to any of numbered paragraphs 10 to 13, wherein the cognitive dysfunction is as defined in numbered paragraph 8 or numbered paragraph 9.
15. A pharmaceutical composition comprising an mGluR modulator, for the treatment, prevention or delay of progression of cognitive dysfunction.
16. A composition according to numbered paragraph 15, wherein the mGluR modulator is as defined in any of numbered paragraphs 2 to 7.
17. A composition according to numbered paragraph 15 or numbered paragraph 16, wherein the mGluR modulator is an mGluR5 modulator
18. A composition according to numbered paragraph 16 or numbered paragraph 17, wherein the mGluR modulator is an mGluR5 antagonist.
19. A kit comprising an mGluR modulator and instructions for using the modulator in the treatment, prevention or delay of progression of cognitive dysfunction.

The following non-limiting Examples illustrate the invention. Further preparations of the mGluR modulators as described herein may be found in other publications, such as , WO 2005/079802, WO 2003/047581, WO 2004/000316, WO 2005/044265, WO 2005/044266, WO 2005/044267, WO 2006/114262 and WO 2007/071358. As such, the following Examples are considered to be non limiting to the other compounds of the invention, A list of abbreviations used is given below.
- AcOH: acetic acid
- BOC: tert-butoxycarbonyl
- n-BuLi: n-butyllithium
- DMF: N,N'-dimethylformamide
- EDC: 1-ethyl-3-[3-(dimethylamino)propyl]-carbodiimide hydrochloride
- HOBt: hydroxybenzotriazole
- AcN: acetonitrile
- BINAP: (2,2'-bis(diphenylphosphino)-1,1'-binaphthyl
- DAST: (Diethylamino)sulfur trifluoride
- DCE: 1,2-dichloroethane
- DCM: dichloromethane
- DIPEA: N,N-diisopropylethylamine
- DMA: N,N-dimethylacetamide
- DMAP: 4-N,N-dimethylaminopyridine
- DME: 1,2-dimethoxyethane
- DMSO: dimethylsulfoxide
- EtOAc: ethylacetate
- ESI: electrospray ionization
- h: hours
- HCI: hydrochloric acid
- HATU: N-[(dimethylamino)-1*H*-1,2,3-triazolo[4,5-b]pyridine-1-ylmethylene]-*N*-methyl- methanaminium hexafluorophosphate *N*³-oxide
- HMPA: hexamethylphosphoramide
- HPLC: high pressure liquid chromatography
- min: minutes
- Mp: melting point
- MS: mass spectroscopy
- MTBE: methyl-*tert*.-butylether
- R_{f}: retention factor (Thin Layer Chromatography)
- rt: room temperature
- t_{R}: retention time
- TFA: trifluoroacetic acid
- THF: tetrahydrofuran

### HPLC specificity

System 1: System 1: Performed on a Waters system equipped with a CTC Analytics HTS PAL autosampler, 515 pumps, and a 996 DAD detector operating at 210 nm. Column: CC70/3 Nucleosil 100-3 C₁₈ (3 µ, 70 x 3 mm, Macherey-Nagel, order #721791.30), temperature: 45°C, flow: 1.2 mL min⁻¹. Eluents: **A**: Water + 0.2% H₃PO₄ (85%, (Merck 100552) + 2% Me₄NOH, (10%, Merck 108123), **B**: Acetonitrile + 20% water + 0.1% H₃PO₄ (85%) + 1% Me₄NOH (10%). Gradient: 0% B to 95% B within 6.6 min., then 95% B 4.4 min.
System 2: Gilson 331 pumps coupled to a Gilson UV/VIS 152 detector and a Finnigan AQA spectrometer (ESI), a 50 µL loop injection valve and a Waters XTerra MS C18 3.5 µm 4.6x50 mm column running a gradient from 5% to 90% acetonitrile containing 0.05% TFA.
System 3: Agilent 1100 Series, LC-MSD and a Agilent Zorbax SB-C18 3x30mm 1.8µm Column running a gradient Water + 0.05% TFA / Acetonitrile + 0.05% TFA from 100/0 to 0/100 over 3.25' - 0/100 over 0.75' - 0/100 to 90/10 over 0.25' with a flux of 0.7 ml/min, 35°C.
System 4: Agilent 1100 Series, LC-MSD and a Agilent Zorbax SB-C18 3x30mm 1.8µm Column running a gradient Water + 0.05% TFA / Acetonitrile + 0.05% TFA from 90/10 to 0/100 over 3.25' - 0/100 over 0.75' - 0/100 to 70/30 over 0.25' with a flux of 0.7 ml/min, 35°C.
System 5: Agilent 1100 Series, LC-MSD and a Agilent Zorbax SB-C18 3x30mm 1.8µm Column running a gradient Water + 0.05% TFA / Acetonitrile + 0.05% TFA from 70/30 to 0/100 over 3.25' - 0/100 over 0.75' - 0/100 to 60/40 over 0.25' with a flux of 0.7 ml/min, 35°C.
System 6: Agilent 1100 Series, LC-MSD and a Agilent Zorbax SB-C18 3x30mm 1.8µm Column running a gradient Water + 0.05% TFA / Acetonitrile + 0.05% TFA from 30/70 to 0/100 over 3.25' - 0/100 over 0.75' - 0/100 to 90/10 over 0.25' with a flux of 0.7 ml/min, 35°C.

### Example 1.1: 6-(4-Chloro-phenylamino)-N,N-diethyl-nicotinamide hydrochloride

6-Chloro-N,N-diethyl-nicotinamide (100 mg, 0.47 mmol) and 4-chloroaniline (184 mg, 1.41 mmol) are suspended in a mixture of glacial acetic acid (0.6 mL) and water (1.4 mL). The reaction mixture is heated in a sealed 3 mL-vial to 100 °C over night. After reaching room temperature the reaction mixture is poured onto MTBE (30 mL) and extracted with 2M HCl (3x 5 mL). The combined acidic extracts are made alkaline by addition of 2M NaOH (10 mL) extracted with MTBE (3x 15 mL). The combined organic extracts are dried (Na₂SO₄) and evaporated to dryness to. The residue is purified by flash-chromatography. To the combined product containing fractions is added 4M HCl in dioxane (0.25 mL) followed by evaporation. The residue is triturated with ether, filtered off, washed with cold ether and vacuum dried at 45 °C to give the title compound as colorless crystals (90 mg, 56%). TLC: R_{f} = 0.16 (MTBE), HPLC: t_{R} = 6.0 min, (system 1); ESI+ MS: m/z = 304.5 (MH⁺).

The starting material can be prepared as described hereafter:

### 6-Chloro-N,N-diethyl-nicotinamide

Under Ar, chloronicotinoyl chloride (4 g, 22 mmol) is suspended in DCM (40 mL). The reaction flask is placed in an ice bath and a solution of diethylamine (2.31 mL, 22 mmol) and triethylamine (3.90 mL, 27.8 mmol) in DCM (40 mL) is added within 45 min keeping the internal temperature below 5 °C. The ice bath is removed and the reaction mixture is stirred for further 30 min. The solution is washed (1x water (40 mL), 1x 1M Na₂CO₃(40mL), 1x water (40 mL)), dried over Na₂SO₄ and evaporated to dryness to afford a reddish orange oil (4.50 g, 95%) which can be used without further purification.

Following the same procedure, the following compounds can be prepared:

### Example 1.2: N,N-Diethyl-6-p-tolylamino-nicotinamide hydrochloride

Yellowish lyophilisate, TLC: R_{f} = 0.22 (MTBE), HPLC: t_{R} = 5.5 min, (system 1); ESI+ MS: m/z = 284.6 (MH⁺).

### Example 1.3: N,N-Diethyl-6-(4-methoxy-phenylamino)-nicotinamide hydrochloride

Light gray crystals, TLC: R_{f} = 0.14 (MTBE), HPLC: t_{R} = 4.6 min, (system 1); ESI+ MS: m/z = 300.6 (MH⁺).

### Example 1.4: 6-(4-Chloro-phenylamino)-N,N-bis-(2-methoxy-ethyl)-nicotinamide hydrochloride

Yellowish lyophilisate, TLC: R_{f} = 0.10 (MTBE), HPLC: t_{R} = 5.6 min, (system 1); ESI+ MS: m/z = 364.5 (MH⁺).

### Example 1.5: [6-(4-Chloro-3-fluoro-phenylamino)-pyridin-3-yl]-piperidin-1-yl-methanone

Colorless crystals, HPLC: t_{R} = 6.6 min, (system 1); ESI+ MS: m/z = 334.5 (MH⁺).

### Example 1.6: [6-(4-Bromo-phenylamino)-pyridin-3-yl]-piperidin-1-yl-methanone

Colorless crystals, TLC: R_{f} = 0.31 (MTBE-ETOAC 9:1), HPLC: t_{R} = 6.3 min, (system 1); ESI+ MS: m/z = 360.6 (MH⁺).

### Example 1.7: 4-[5-(Piperidine-1-carbonyl)-pyridin-2-ylamino]-benzonitrile

Colorless crystals, TLC: R_{f} = 0.14 (MTBE), HPLC: t_{R} = 5.7 min, (system 1); ESI+ MS: m/z = 307.6 (MH⁺).

### Example 1.8: Piperidin-1-yl-[6-(4-trifluoromethoxy-phenylamino)-pyridin-3-yl]-methanone

Colorless crystals, TLC: R_{f} = 0.29 (DCM-ETOAC 7:3), HPLC: t_{R} = 6.6 min, (system 1); ESI+ MS: m/z = 366.7 (MH⁺).

### Example 1.9: [6-(4-Chloro-phenylamino)-pyridin-3-yl]-(2-methyl-piperidin-1-yl)-methanone hydrochloride

TLC: R_{f} = 0.23 (MTBE), HPLC: t_{R} = 6.5 min, (system 1); ESI+ MS: m/z = 330.5 (MH⁺).

### Example 1.10: (2-Methyl-piperidin-1-yl)-(6-p-tolylamino-pyridin-3-yl)-methanone

Beige crystals, TLC: R_{f} = 0.24 (MTBE), HPLC: t_{R} = 6.0 min, (system 1); ESI+ MS: m/z = 310.5 (MH⁺).

### Example 1.11: [6-(4-Methoxy-phenylamino)-pyridin-3-yl]-(2-methyl-piperidin-1-yl)-methanone hydrochloride

Purple crystals, TLC: R_{f} = 0.27 (MTBE), HPLC: t_{R} = 5.4 min, (system 1); ESI+ MS: m/z = 326.5 (MH⁺).

### Example 1.12: rac-[6-(4-Chloro-phenylamino)-pyridin-3-yl]-(3-methyl-piperidin-1-yl)-methanone

Colorless crystals, TLC: R_{f} = 0.25 (MTBE), HPLC: t_{R} = 6.6 min, (system 1); ESI+ MS: m/z = 330.5 (MH⁺).

Using either S-3-methylpiperidine or R-3-methylpiperidine as starting material the pure enantiomers could be prepared:

### [6-(4-Chloro-phenylamino)-pyridin-3-yl]-(S-3-methyl-piperidin-1-yl)-methanone

Colorless crystals, TLC: R_{f}, = 0.22 (MTBE), HPLC: t_{R} = 6.7 min, (system 1); ESI+ MS: m/z = 330.1 (MH⁺).

### [6-(4-Chloro-phenylamino)-pyridin-3-yl]-(R-3-methyl-piperidin-1-yl)-methanone

Beige crystals, HPLC: t_{R} = 6.7 min, (system 1); ESI+ MS: m/z = 330.2 (MH⁺).

### Example 1.13: 3-Methyl-piperidin-1-yl)-(6-p-tolylamino-pyridin-3-yl)-methanone

Pink lyophilisate, HPLC: t_{R} = 6.2 min, (system 1); ESI+ MS: m/z = 310.5 (MH⁺).

### Example 1.14: [6-(4-Methoxy-phenylamino)-pyridin-3-yl]-(3-methyl-piperidin-1-yl)-methanone hydrochloride

Brown crystals, HPLC: t_{R} = 5.6 min, (system 1); ESI+ MS: m/z = 326.5 (MH⁺).

### Example 1.15: (3-Methyl-piperidin-1-yl)-(6-phenylamino-pyridin-3-yl)-methanone hydrochloride

Colorless crystals, TLC: R_{f} = 0.26 (MTBE), HPLC: t_{R} = 5.8 min, (system 1); ESI+ MS: m/z = 296.5 (MH⁺).

### Example 1.16 [6-(3-Chloro-phenylamino)-pyridin-3-yl]-(3-methyl-piperidin-1-yl)-methanone hydrochloride

TLC: R_{f} = 0.27 (MTBE), HPLC: t_{R} = 6.6 min, (system 1); ESI+ MS: *m*/*z* = 330.5 (MH⁺).

### Example 1.17: [6-(4-Chloro-phenylamino)-pyridin-3-yl]-morpholin-4-yl-methanone hydrochloride

Yellowish crystals, TLC: R_{f} = 0.38 (MTBE-MeOH 9:1), HPLC: t_{R} = 5.5 min, (system 1); ESI+ MS: m/z = 318.5 (MH⁺).

### Example 1.18: [6-(4-Methoxy-phenylamino)-pyridin-3-yl]-morpholin-4-yl-methanone hydrochloride

Greenish solid, TLC: R_{f} = 0.35 (MTBE-MeOH 9:1), HPLC: t_{R} = 4.0 min, (system 1); ESI+ MS: m/z = 314.5 (MH⁺).

### Example 1.19: cis-[6-(4-Chloro-phenylamino)-pyridin-3--yl]-(2,6-dimethyl-morpholin-4-yl)-methanone hydrochloride

Colorless crystals, TLC: R_{f} = 0.13 (MTBE), HPLC: t_{R} = 6.1 min, (system 1); ESI+ MS: m/z = 346.5 (MH⁺).

### Example 1.20: (cis-2,6-Dimethyl-morpholin-4-yl)-(6-p-tolylamino-pyridin-3-yl)-methanone hydrochloride

Beige crystals, TLC: R_{f} = 0.24 (MTBE), HPLC: t_{R} = 5.4 min, (system 1); ESI+ MS: m/z = 326.6 (MH⁺).

### Example 1.21: (cis-2,6-Dimethyl-morpholin-4-yl)-[6-(4-methoxy-phenylamino)-pyridin-3-yl]-methanone hydrochloride

Purple crystals, TLC: R_{f} = 0.16 (MTBE), HPLC: t_{R} = 4.9 min, (system 1); ESI+ MS: m/z = 342.5 (MH⁺).

### Example 2.1: [6-(5-Chloro-pyridin-2-ylamino)-pyridin-3-yl]-piperidin-1-yl-methanone

A solution of palladium(II) acetate (2 mg, 9 µmol) and BINAP (5.6 mg, 9 µmol) in dry and degassed toluene (1.5 mL) is stirred for 10 min under Ar. Then, the clear yellow solution obtained is added to a degassed suspension of (6-chloro-pyridin-3-yl)-piperidin-1-yl-methanone (100 mg, 0.45 mmol, prepared according to the general procedure stated in example 1.1), 2-amino-5-chloropyridine (70 mg, 0.53 mmol), and KO*t*Bu (257 mg, 2.22 mmol) in dry toluene. The reaction mixture is stirred for 5 h in a sealed 5 mL-vial. After reaching room temperature the mixture is poured into MTBE (30 mL), washed (3x H₂O (20 mL)), dried over Na₂SO₄ and evaporated to give a turbid oil. Crystallization from Et₂O affords the title compound as beige crystals (87 mg, 62%), HPLC: t_{R} = 4.8 min, (system 1); ESI+ MS: m/z = 317.6 (MH⁺).

Following the same procedure, the following compounds can be prepared:

### Example 2.2: Azepan-1-yl-[6-(pyridin-3-ylamino)-pyridin-3-yl]-methanone

Yellowish lyophilisate, TLC: R_{f} = 0.28 (MTBE-MeOH 85:15), HPLC: t_{R} = 4.2 min, (system 1); ESI+ MS: m/z = 297.2 (MH⁺).

### Example 2.3: [6-(3,4-Difluoro-phenylamino)-pyridin-3-yl]-piperidin-1-yl-methanone

Colorless crystals, HPLC: t_{R} = 6.1 min, (system 1); ESI+ MS: m/z = 318.6 (MH⁺).

### Example 2.4: rac-(2-Aza-bicyclo[2.2.1]hept-2-yl)-[5-chloro-6-(4-chloro-phenylamino)-pyridin-3-yl]-methanone

Beige powder, HPLC: t_{R} = 6.9 min, (system 1); ESI+ MS: m/z = 364.0 (MH⁺).

### Example 2.5: [5-Chloro-6-(4-chloro-phenylamino)-pyridin-3-yl]-thiomorpholin-4-yl-methanone

Beige powder, HPLC: t_{R} = 6.6 min, (system 1); ESI+ MS: m/z = 370.0 (MH⁺).

### Example 2.6: rac-[5-Chloro-6-(6-methoxy-pyridin-3-ylamino)-pyridin-3-yl]-(3-methyl-piperidin-1-yl)-methanone

Beige lyophilisate, TLC: R_{f} = 0.49 (MTBE), HPLC: t_{R} = 6.2 min (system 1); ESI+ MS: m/z = 361.1 (MH⁺).

### Example 2.7: Azepan-1-yl-[5-chloro-6-(6-methoxy-pyridin-3-ylamino)-pyridin-3-yl]-methanone

Beige lyophilisate, TLC: R_{f} = 0.32 (MTBE), HPLC: t_{R} = 6.0 min (system 1); ESI+ MS: m/z = 361.1 (MH⁺).

### Example 2.8: [5-Chloro-6-(6-methoxy-pyridin-3-ylamino)-pyridin-3-yl]-piperidin-1-yl-methanone

Colorless lyophilisate, TLC: R_{f} = 0.36 (MTBE), HPLC: t_{R} = 5.9 min (system 1); ESI+ MS: m/z = 347.0 (MH⁺).

### Example 2.9: [5-Chloro-6-(6-ethoxy-pyridin-3-ylamino)-pyridin-3-yl]-piperidin-1-yl-methanone

Colorless lyophilisate, TLC: R_{f} = 0.23 (EtOAc/hexanes 1:1), LC/MS: m/z = 361 (MH⁺).

### Example 2.10: rac-[5-Chloro-6-(6-methyl-pyridin-3-ylamino)-pyridin-3-yl]-(3-methyl-piperidin-1-yl)-methanone

Beige crystals, HPLC: t_{R} = 4.7 min (system 1); ESI+ MS: m/z = 345.1 (MH⁺).

Using either S-3-methylpiperidine or R-3-methylpiperidine as starting material the pure enantiomers could be prepared:

### Example 2.10a: [5-Chloro-6-(6-methyl-pyridin-3-ylamino)-pyridin-3-yl]-(S-3-methyl-piperidin-1-yl)-methanone

Brown gum, HPLC: t_{R} = 4.7 min (system 1); ESI+ MS. m/z = 345.1 (MH⁺)

### Example 2.10b: [5-Chloro-6-(6-methyl-pyridin-3-ylamino)-pyridin-3-yl]-(R-3-methyl-piperidin-1-yl)-methanone

Brown gum, HPLC: t_{R} = 4.5 min (system 1); ESI+ MS. m/z = 345.1 (MH⁺)

### Example 2.11: [5-Chloro-6-(6-methyl-pyridin-3-ylamino)-pyridin-3-yl]-piperidin-1-yl-methanone

Colorless crystals, HPLC: t_{R} = 4.3 min (system 1); ESI+ MS: m/z = 331.1 (MH⁺).

### Example 2.12: Azepan-1-yl-[5-chloro-6-(6-methyl-pyridin-3-ylamino)-pyridin-3-yl]-methanone

Colorless crystals, HPLC: t_{R} = 4.3 min (system 1); ESI+ MS: m/z = 345.1 (MH⁺).

### Example 2.13: rac-(2-Aza-bicyclo[2.2.1]hept-2-yl)-[5-chloro-6-(6-methyl-pyridin-3-ylamino)-pyridin-3-yl]-methanone

Beige powder, HPLC: t_{R} = 4.1 min (system 1); ESI+ MS: m/z = 343.1 (MH⁺).

### Example 2.14: [5-Chloro-6-(6-methyl-pyridin-3-ylamino)-pyridin-3-yl]-thiazolidin-3-yl-methanone

Beige powder, HPLC: t_{R} = 4.1 min (system 1); ESI+ MS: m/z = 335.0 (MH⁺).

### Example 2.15: [5-Chloro-6-(6-methyl-pyridin-3-ylamino)-pyridin-3-yl]-thiomorpholin-4-yl-methanone

Beige powder, HPLC: t_{R} = 3.9 min (system 1); ESI+ MS: m/z = 349.0 (MH⁺).

### Example 2.16: [5-Chloro-6-(2-methyl-pyrimidin-5-ylamino)-pyridin-3-yl]-(3-methyl-piperidin-1-yl)-methanone

Colorless crystals, HPLC: t_{R} = 5.6 min (system 1); ESI+ MS: m/z = 346.1 (MH⁺).

### Example 2.17: [5-Chloro-6-(2-methyl-pyrimidin-5-ylamino)-pyridin-3-yl]-piperidin-1-yl-methanone

Colorless crystals, HPLC: t_{R} = 5.1 min (system 1); ESI+ MS: m/z = 332.1 (MH⁺).

### Example 2.18: Azepan-1-yl-[5-chloro-6-(2-methyl-pyrimidin-5-ylamino)-pyridin-3-yl]-methanone

Colorless crystals, HPLC: t_{R} = 5.5 min (system 1); ESI+ MS: m/z = 346.1 (MH⁺).

### Example 2.19: [5-Chloro-6-(6-methyl-pyridin-3-ylamino)-pyridin-3-yl]-(3-ethyl-piperidin-1-yl)-methanone

A mixture of (5,6-dichloro-pyridin-3-yl)-(3-ethyl-piperidin-1-yl)-methanone (300 mg, 1.04 mmol), 3-amino-6-methyl pyridine (171 mg, 1.57 mmol), Pd(OAc)₂ (7 mg, 0.03 mmol), rac-BINAP (20 mg, 0.03 mmol) and potassium carbonate (723 mg, 5.2 mmol) in degassed toluene (10 mL) was stirred, under argon, at 80°C for 3 hours. EtOAc was added and the organic phase was washed with water, dried over sodium sulfate and concentrated in vacuo to give a crude beige powder. The crude material was sonicated in pentane/Et₂O and then filtered. After high-vacuum drying, [5-chloro-6-(6-methyl-pyridin-3-ylamino)-pyridin-3-yl]-(3-ethyl-piperidin-1-yl)-methanone (100 mg, 27%) was obtained as a beige powder. (ES-MS: *m*/*z* 359.3/361.3 [M+H]⁺, t_{R} 3.52 min (system 2)).

The starting material was prepared as described hereafter:
i) 3-ethyl piperidine
   3-Ethyl pyridine (5.0 g, 46.7 mmol) was hydrogenated in AcOH (100 mL) over PtO₂ (500 mg) under 4 bar for 4 hours. The mixture was filtered through a pad of celite and washed with AcOH. The solvent was removed in vacuo and the residue was dissolved into water. The solution was basified by addition with 40% NaOH solution. The aqueous phase was extracted with Et₂O. The organic phases were combined, dried over sodium sulfate and concentrated in vacuo to afford 3-ethyl piperidine (4.4 g, 83%) as a clear yellow oil.
ii) (5,6-Dichloro-pyridin-3-yl)-(3-ethyl-piperidin-1-yl)-methanone
   A mixture of 5,6 dichloronicotinic acid (1 g, 5.2 mmol) in SOCl₂ (6 mL) was stirred at 70°C for 4 hours. The solvent was removed in vacuo to give a beige oil (1.05 g) corresponding to the acid chloride. This oil was solublised in DCM (15 mL) and at 0°C triethylamine (1.1 mL, 7.84 mmol) was added. Then, a solution of 3-ethyl piperidine (657 mg, 5.75 mmol) in DCM (5 mL) was added carefully drop-wise. At the end of the addition, the mixture was stirred at RT for 30 min. Water was added and the aqueous phase was extracted with DCM. The organic phases were combined, dried over sodium sulfate and concentrated in vacuo to (5,6-dichloro-pyridin-3-yl)-(3-ethyl-piperidin-1-yl)-methanone (1.2 g, 80%) as a yellow oil. (ES-MS: *m*/*z* 328.2/330.2 [M+CH₃CN+ H]⁺, t_{R} 5.48 min (system 2)).

### Example 2.20: [5-Chloro-6-(6-methoxy-pyridin-3-ylamino)-pyridin-3-yl]-(3-ethyl-piperidin-1-yl)-methanone

A mixture of (5,6-dichloro-pyridin-3-yl)-(3-ethyl-piperidin-1-yl)-methanone (300 mg, 1.04 mmol), 5-amino-2-methoxy pyridine (201 mg, 1.57 mmol), Pd(OAc)₂ (7 mg, 0.03 mmol), rac-BINAP (20 mg, 0.03 mmol) and potassium carbonate (723 mg, 5.2 mmol) in degassed toluene (10 mL) was stirred, under argon, at 80°C for 3 hours. EtOAc was added and the organic phase was washed with water, dried over sodium sulfate and concentrated in vacuo to give a crude beige powder. The crude material was purified by flash chromatography using EtOAc/Hexanes as eluent to afford [5-chloro-6-(6-methoxy-pyridin-3-ylamino)-pyridin-3-yl]-(3-ethyl-piperidin-1-yl)-methanone (60 mg, 15%) as a beige powder. (ES-MS: *m*/*z* 375.3/375.5 [M+H]⁺, t_{R} 5.21 min (system 2))

### Example 2.21: [5-Chloro-6-(4-chloro-phenylamino)-pyridin-3-yl]-(3-ethyl-piperidin-1-yl)-methanone

A mixture of (5,6-dichloro-pyridin-3-yl)-(3-ethyl-piperidin-1-yl)-methanone (300 mg, 1.04 mmol), 4-chloro aniline (206 mg, 1.57 mmol), Pd(OAc)₂ (7 mg, 0.03 mmol), rac-BINAP (20 mg, 0.03 mmol) and potassium carbonate (723 mg, 5.2 mmol) in degassed toluene (10 mL) was stirred, under argon, at 80°C for 3 hours. EtOAc was added and the organic phase was washed with water, dried over sodium sulfate and concentrated in vacuo to give a crude beige powder. The crude material was purified by flash chromatography using EtOAc/Hexanes as eluent to afford [5-chloro-6-(4-chloro-phenylamino)-pyridin-3-yl]-(3-ethyl-piperidin-1-yl)-methanone (150 mg, 38%) as a beige powder. (ES-MS: m/z 378.2/380.3 [M+H]⁺, t_{R} 6.50 min (system 2))

### Example 2.22: [5-Chloro-6-(6-methyl-pyridin-3-ylamino)-pyridin-3-yl]-(3-propyl-piperidin-1-yl)-methanone

A mixture of (5,6-dichloro-pyridin-3-yl)-(3-propyl-piperidin-1-yl)-methanone (440 mg, 1.26 mmol), 3-amino-6-methyl pyridine (210 mg, 1.88 mmol), Pd(OAc)₂ (8.6 mg, 0.03 mmol), rac-BINAP (24 mg, 0.03 mmol) and potassium carbonate (879 mg, 5.0 mmol) in degassed toluene (10 mL) was stirred, under argon, at 80°C for 3 hours. EtOAc was added and the organic phase was washed with water, dried over sodium sulfate and concentrated in vacuo to give a crude beige powder. The crude material was purified by flash chromatography using EtOAc/Hexanes as eluent to afford [5-chloro-6-(6-methyl-pyridin-3-ylamino)-pyridin-3-yl]-(3-propyl-piperidin-1-yl)-methanone (110 mg, 23%) as a beige powder. (ES-MS: *m*/*z* 373.3/375.3 [M+H]⁺, t_{R} 4.52 min (system 2))

The starting material was prepared as described hereafter:
i) 3-propyl pyridine
   At 0°C, to a mixture of diisopropylamine (3.53 mL, 24.7 mmol) in THF (35 mL), BuLi (1.6 M in hexanes, 15.4 mL, 25 mmol) was added drop-wise. After 30 min, HMPA (15.7 g, 24.7 mmol) was aded and the mixture was kept at 0°C for 15 min. Then a solution of 3-methyl pyridine (2.3 g, 24.7 mmol) in THF (10 mL) was added drop-wise. After 30 min, Etl (3.45 g, 24.7 mmol) in THF (10 mL) was added drop-wise and the mixture was then stirred at RT for 1 hour. The mixture was poured into 10% HCl. The aqueous phase was extracted with Et₂O. The organic phase was washed with water, dried over sodium sulfate and concentrated in vacuo to afford a yellow oil (300 mg, 10%) which will be used without further purification.
ii) 3-propyl piperidine
   3-propyl pyridine (300 mg, 2.48 mmol) was hydrogenated in AcOH (20 mL) over PtO₂ (50 mg) under 4 bar for 16 hours. The mixture was filtered through a pad of celite and washed with AcOH. The solvent was removed in vacuo and the residue was dissolved into water. The solution was basified by addition with 40% NaOH solution. The aqueous phase was extracted with Et₂O. The organic phases were combined, dried over sodium sulfate and concentrated in vacuo to afford 3-propyl piperidine (300 mg, 95%) as a clear yellow oil.
iii) (5,6-Dichloro-pyridin-3-yl)-(3-propyl-piperidin-1-yl)-methanone
   5,6-Dichloro-nicotinoyl chloride (550 mg, 2.61 mmol) was solubilised in DCM (15 mL) and at 0°C triethylamine (0.54 mL, 3.95 mmol) was added. Then, a solution of 3-propyl piperidine (369 mg, 2.87 mmol) in DCM (5 mL) was added carefully drop-wise. At the end of the addition, the mixture was stirred at RT for 30 min. Water was added and the aqueous phase was extracted with DCM. The organic phases were combined, dried over sodium sulfate and concentrated in vacuo to afford a beige-brown oil. This oil was sonicated in pentane to afford (5,6-dichloro-pyridin-3-yl)-(3-propyl-piperidin-1-yl)-methanone (440 mg, 48%) as a beige-brown solid.

### Example 2.23: [5-Chloro-6-(6-trifluoromethyl-pyridin-3-ylamino)-pyridin-3-yl]-((R)-2-ethyl-piperidin-1-yl)-methanone

LC/MS: m/z = 413 (MH⁺); TLC: R_{f} = 0.40 (DCM/MeOH 95:5).

### Example 2.24: [5-Chloro-6-(6-methoxy-pyridin-3-ylamino)-pyridin-3-yl]-((R)-2-ethyl-piperidin-1-yl)-methanone

LC/MS: m/z = 375 (MH⁺); TLC: R_{f} = 0.40 (DCM/MeOH 95:5).

### Example 3.1: rac-[5-Chloro-6-(6-methyl-pyridin-3-yloxy)-pyridin-3-yl]-(3-methyl-piperidin-1-yl)-methanone

To a solution of rac-(5,6-dichloropyridin-3-yl)-(3-methyl-piperidin-1-yl)-methanone (50 mg, 0.18 mmol, prepared according to the procedure stated in example 1.1) and 4-chlorophenol (23.5 mg, 0.18 mmol) in dry DMA (1 mL) is added finely ground anhydrous K₂CO₃ (50.6 mg, 0.36 mmol). The suspension is microwave heated to 140 °C in a sealed 5 mL-vial for 45 min with stirring. Then, the reaction mixture is diluted with ethyl acetate (10 mL) and washed with brine (10 mL). The organic layer is dried (Na₂SO₄) and evaporated to dryness to give a brown oil. Purification by preparative HPLC afforded the title compound as colorless syrup (40 mg, 60%), HPLC: t_{R} = 7.1 min (system 1); ESI+ MS: m/z = 365.0 (MH⁺).

Following the same procedure, the following compounds can be prepared:

### Example 3.2: [5-Chloro-6-(6-methyl-pyridin-3-yloxy)-pyridin-3-yl]-piperidin-1-yl-methanone

Colorless syrup, HPLC: t_{R} = 6.8 min (system 1); ESI+ MS: m/z = 351.0 (MH⁺).

### Example 3.3: Azepan-1-yl-[5-chloro-6-(6-methyl-pyridin-3-yloxy)-pyridin-3-yl]-methanone

Colorless syrup, HPLC: t_{R} = 7.0 min (system 1); ESI+ MS: m/z = 365.0 (MH⁺).

### Example 4.1: [6-(6-Methyl-pyridin-3-ylamino)-pyridin-3-yl]-piperidin-1-yl-methanone

To 6-(6-methyl-pyridin-3-ylamino)-nicotinic acid (210 mg, 0.92 mmol) is added thionyl chloride (2 mL). The colorless suspension is refluxed under argon for 20 min. After cooling the excess thionyl chloride is stripped off. The residue is redissolved in DCM (6 mL) and a solution of piperidine (0.11 mL, 1.10 mmol) and triethylamine (1.28 mL, 9.16 mmol) in DCM (2 mL) is quickly added. The yellow slightly turbid solution is stirred for 20 min at room temperature. Then, MTBE (60 mL) is added and the solution is extracted twice with water and brine. The organic layer is dried over Na₂SO₄ and evaporated to give a yellow foam. Flash chromatography (20 g silica gel, MeOH-MTBE gradient 2% -> 15% MeOH, flow 20 mL min⁻¹) followed by crystallization from ether affords the title compound as colorless crystals (573 mg, 63%), TLC: R_{f} = 0.18 (MTBE-MeOH 9:1), HPLC: t_{R} = 3.8 min (system 1); ESI+ MS: m/z = 297.5 (MH⁺).

The starting material can be prepared as described hereafter:
i) *Methyl 6-(6-Methyl-pyridin-3-ylamino)-nicotinate*
   To 5-amino-2-methylpyridine (2.22 g, 20.56 mmol) and finely ground anhydrous K₂CO₃ (11.9 g, 85.2 mmol) is added dry toluene (30 mL) under argon. Then, a solution of palladium(II) acetate (79 mg, 0.34 mmol) and BINAP (218 mg, 0.34 mmol) in dry toluene (10 mL) is added. The reaction mixture is placed in an oil bath (70 °C) and a solution of methyl 6-chloronicotinate (3.0 g, 17.1 mmol) in dry toluene (20 mL) is added dropwise within 30 min. After 1.5 h the oilbath is removed and the reaction flask is placed in an ice bath. After stirring for 15 min the product is filtered off. The filter cake is triturated three times with THF / MeOH 1:1 (100 mL). The combined extracts are evaporated to dryness to give a brown powder. Flash chromatography (gradient MTBE-MeOH 100:0 - MTBE-MeOH 85:15) followed by crystallization from ether gives the product as light pink crystals (1.86 g, 45%).
ii) *6-(6-Methyl-pyridin-3-ylamino)-nicotinic acid*
   To a suspension of methyl 6-(6-Methyl-pyridin-3-ylamino)-nicotinate (2.72 g, 11.18 mmol) in methanol (55 mL) is added 2M NaOH (17 mL). The reaction mixture is heated to 60 °C for 30 min. After 15 min a clear reddish solution is formed. Then, the reaction flask is placed in an ice bath and 2M HCl (17 mL) is added at such a rate that the internal temperature does not exceed 20°C. After evaporation of methanol the suspension is diluted with water (50 mL). The product is filtered off, washed with cold water and vacuum dried at 60°C over night to give a pink powder (2.78 g, 100%).

Following the same procedure, the following compounds can be prepared:

### Example 4.2: Azepan-1-yl-[6-(4-chloro-phenylamino)-pyridin-3-yl]-methanone

Yellow foam, TLC: R_{f} = 0.25 (MTBE), HPLC: t_{R} = 6.5 min (system 1); ESI+ MS: m/z = 330.5 (MH⁺).

### Example 4.3: [6-(4-Chloro-phenylamino)-pyridin-3-yl]-(3,3-difluoro-piperidin-1-yl)-methanone

Colorless crystals, TLC: R_{f} = 0.23 (MTBE), HPLC: t_{R} = 6.1 min (system 1); ESI+ MS: m/z = 352.6 (MH⁺).

### Example 4.4: [6-(4-Chloro-phenylamino)-pyridin-3-yl]-(4-methyl-piperidin-1-yl)-methanone

Colorless crystals, TLC: R_{f} = 0.3 (MTBE), HPLC: t_{R} = 6.6 min (system 1); ESI+ MS: m/z = 330.6 (MH⁺).

### Example 4.5: [6-(4-Chloro-phenylamino)-pyridin-3-yl]-(3,5-dimethyl-piperidin-1-yl)-methanone (diastereomeric mixture cis / trans 72:28)

Colorless crystals, TLC: R_{f} = 0.35 (MTBE), HPLC: t_{R} = 6.9 min (trans diastereomer, 28%), 7.0 min (cis diastereomer, 72%) (system 1); ESI+ MS: m/z = 344.6 (MH⁺).

### Example 4.6: rac-[6-(4-Chloro-phenylamino)-pyridin-3-yl]-(3-hydroxymethyl-piperidin-1-yl)-methanone

Colorless foam, TLC: R_{f} = 0.32 (MTBE-MeOH 9:1), HPLC: t_{R} = 5.2 min (system 1); ESI+ MS: m/z = 346.5 (MH⁺).

### Example 4.7: rac-[6-(4-Chloro-phenylamino)-pyridin-3-yl]-(3-methoxy-piperidin-1-yl)-methanone

Colorless foam, TLC: R_{f} = 0.43 (MTBE-MeOH 9:1), HPLC: t_{R} = 5.8 min (system 1); ESI+ MS: m/z = 346.5 (MH⁺).

### Example 4.8: [6-(4-Chloro-phenylamino)-pyridin-3-yl]-(octahydro-quinolin-1-yl)-meth anone (diastereomeric mixture, cis / trans)

Colorless foam, TLC: R_{f} = 0.22, 0.29 (MTBE-MeOH 9:1), HPLC: t_{R} = 7.3 min (system 1); ESI+ MS: m/z = 370.7 (MH⁺).

### Example 4.9: (3-Aza-bicyclo[3.2.2]non-3-yl)-[6-(4-chloro-phenylamino)-pyridin-3-yl]-methanone

Foam, TLC: R_{f} = 0.28 (MTBE), HPLC: t_{R} = 6.9 min (system 1); ESI+ MS: m/z = 356.6 (MH⁺).

### Example 4.10: (2-Aza-tricyclo[3.3.1.1*3,7*]dec-2-yl-[6-(4-ch/oro-phenylamino)-pyridin-3-y/]-methanone

Colorless crystals, TLC: R_{f} = 0.23 (MTBE), HPLC: t_{R} = 7.0 min (system 1); ESI+ MS: m/z = 368.6 (MH⁺).

### Example 4.11: [6-(4-Chloro-phenylamino)-pyridin-3-yl]-(3-hydroxy-8-aza-bicyclo[3.2.1]oct-8-yl)-methanone

Colorless foam, TLC: R_{f} = 0.36 (MTBE-MeOH 9:1), HPLC: t_{R} = 5.4 min (system 1); ESI+ MS: m/z = 358.6 (MH⁺).

### Example 4.12: rac-(2-Aza-bicyclo[2.2.1]hept-2-yl)-[6-(4-chloro-phenylamino)-pyridin-3-yl]-methanone

Colorless crystals, TLC: R_{f} = 0.31 (MTBE-MeOH 95:5), HPLC: t_{R} = 6.2 min (system 1); ESI+ MS: m/z = 328.6 (MH⁺).

### Example 4.13: rac-(3-Methyl-piperidin-1-yl)-[6-(6-methyl-pyridin-3-ylamino)-pyridin-3-yl]-methanone

Yellow foam, TLC: R_{f} = 0.26 (MTBE-MeOH 9:1), HPLC: t_{R} = 4.4 min (system 1); ESI+ MS: m/z = 311.6 (MH⁺).

Using either S-3-methylpiperidine or R-3-methylpiperidine as starting material the pure enantiomers could be prepared:

### Example 4.13a: (S-3-Methyl-piperidin-1-yl)-[6-(6-methyl-pyridin-3-ylamino)-pyridin-3-yl]-methanone

Colorless foam, TLC: R_{f} = 0.32 (MTBE-MeOH 85:15), HPLC: t_{R} = 4.1 min (system 1); ESI+ MS: m/z = 311.2 (MH⁺).

### Example 4.13b: (R-3-Methyl-piperidin-1-yl)-[6-(6-methyl-pyridin-3-ylamino)-pyridin-3-yl]-methanone

Colorless foam, HPLC: t_{R} = 4.1 min (system 1); ESI+ MS: m/z = 311.2 (MH⁺).

### Example 4.14: [6-(4-Chloro-phenylamino)-pyridin-3-yl]-(rel-(3aR, 4S, 7aR)-4-hydroxy-4-m-tolylethynyl-octahydro-indol-1-yl)-methanone

Yellow foam, TLC: R_{f} = 0.32 (MTBE-MeOH 95:5), HPLC: t_{R} = 6.8 min (system 1); ESI+ MS: m/z = 486.7 (MH⁺).

### Example 4.15: Azepan-1-yl-[6-(6-methyl-pyridin-3-ylamino)-pyridin-3-yl]-methanone

Yellow crystals, TLC: R_{f} = 0.2 (MTBE-MeOH 9:1), HPLC: t_{R} = 4.0 min (system 1); ESI+ MS: m/z = 311.6 (MH⁺).

### Example 4.16: Azocan-1-yl-[6-(6-methyl-pyridin-3-ylamino)-pyridin-3-yl]-methanone

TLC: R_{f} = 0.33 (MTBE-MeOH 85:15), HPLC: t_{R} = 4.5 min (system 1); ESI+ MS: m/z = 325.6 (MH⁺).

### Example 4.17: [5-Chloro-6-(6-methyl-pyridin-3-ylamino)-pyridin-3-yl]-(2-ethyl-piperidin-1-yl)-methanone

TLC: R_{f} = 0.13 (DCM/MeOH 95:5), HPLC: t_{R} = 2.8 min (system 4); LC/MS MS: m/z = 359 (MH⁺).

### Example 4.18: [5-Chloro-6-(6-methyl-pyridin-3-ylamino)-pyridin-3-yl]-((R)-2-ethyl-piperidin-1-yl)-methanone

TLC: R_{f} = 0.81 (DCM/MeOH 5:1), HPLC: t_{R} = 2.8 min (system 4); LC/MS MS: m/z = 359 (MH⁺); [α]_{D} = -33.6° (c=1.0, CHCl₃, 20 °C).

### Example 4.19: [5-Chloro-6-(6-methyl-pyridin-3-ylamino)-pyridin-3-yl]-((S)-2-ethyl-piperidin-1-yl)-methanone

TLC: R_{f} = 0.81 (DCM/MeOH 95:5), HPLC: t_{R} = 2.8 min (system 4); LC/MS MS: m/z = 359 (MH⁺), [α]_{Hg578} = +1.64° (c=0.16, DCM, 20 °C).

### Example 4.20: [5-Chloro-6-(6-methyl-pyridin-3-ylamino)-pyridin-3-yl]-(2,3-dimethyl-piperidin-1-yl)-methanone

TLC: R_{f} = 0.75 (DCM/MeOH 5:1), HPLC: t_{R} = 2.8 min (system 4); LC/MS MS: m/z = 359 (MH⁺).

### Example 4.21: [5-Chloro-6-(6-methyl-pyridin-3-ylamino)-pyridin-3-yl]-((2S,3S)-2,3-dimethyl-piperidin-1-yl)-methanone

TLC: R_{f} = 0.66 (DCM/MeOH 5:1), HPLC: t_{R} = 2.76 min (system 4); LC/MS MS: m/z = 359 (MH⁺), [α]_{Hg578}= +0.9° (c=0.11, DCM, 20 °C).

### Example 4.22: [5-Chloro-6-(6-methyl-pyridin-3-ylamino)-pyridin-3-yl]-((2R,3R)-2,3-dimethyl-piperidin-1-yl)-methanone

TLC: R_{f} = 0.72 (DCM/MeOH 5:1), HPLC: t_{R} = 2.76 min (system 4); LC/MS MS: m/z = 359 (MH⁺), [α]_{Hg578}=-1.0° (c=0.11, DCM, 20 °C).

### Example 4.23: (5-Chloro-6-(6-methyl-pyridin-3-ylamino)-pyridin-3-yl]-((S)-2-methyl-piperidin-1-yl)-methanone

TLC: R_{f} = 0.87 (DCM/MeOH 5:1), HPLC: t_{R} = 2.65 min (system 4); LC/MS MS: m/z = 345 (MH⁺), [α]_{Hg578} = +0.10° (c=0.67, DCM, 20 °C).

### Example 4.24: [5-Chloro-6-(6-methyl-pyridin-3-ylamino)-pyridin-3-yl]-((R)-2-methyl-piperidin-1-yl)-methanone

TLC: R_{f} = 0.80 (DCM/MeOH 5:1), HPLC: t_{R} = 2.65 min (system 4); LC/MS MS: m/z = 345 (MH⁺), [α]_{Hg578}= -0.10° (c=0.67, DCM, 20 °C).

### Example 4.25: [5-Chloro-6-(6-methyl-pyridin-3-ylamino)-pyridin-3-yl]-(octahydro-[1]pyrindin-1-yl)-methanone

TLC: R_{f} = 0.79 (DCM/MeOH 5:1), HPLC: t_{R} = 2.76 min (system 4); LC/MS MS: m/z = 371 (MH⁺).

### Example 4.26: [5-Chloro-6-(6-methyl-pyridin-3-ylamino)-pyridin-3-yl]-(4aS,7aS)-octahydro-[1]pyrindin-1-yl-methanone

TLC: R_{f} = 0.64 (DCM/MeOH 5:1), HPLC: t_{R} = 2.86 min (system 4); LC/MS MS: m/z = 371 (MH⁺), [α]_{Hg578} = +0.12° (c=0.007, DCM, 20 °C).

### Example 4.27: [5-Chloro-6-(6-methyl-pyridin-3-ylamino)-pyridin-3-yl]-(4aR,7aR)-octahydro-[1]pyrindin-1-yl-methanone

TLC: R_{f} = 0.64 (DCM/MeOH 5:1), HPLC: t_{R} = 2.84 min (system 4); LC/MS MS: m/z = 371 (MH⁺), [α]_{Hg578} = -0.15° (c=0.007, DCM, 20 °C).

### Example 4.28: [5-Chloro-6-(6-methyl-pyridin-3-ylamino)-pyridin-3-yl]-(2-isopropyl-piperidin-1-yl)-methanone

TLC: R_{f} = 0.76 (DCM/MeOH 5:1), HPLC: t_{R} = 2.90 min (system 4); LC/MS MS: m/z = 373 (MH⁺).

### Example 4.29: [5-Chloro-6-(6-methyl-pyridin-3-ylamino)-pyridin-3-y/]-((R)-2-isopropyl-piperidin-1-yl)-methanone

TLC: R_{f} = 0.63 (DCM/MeOH 5:1), HPLC: t_{R} = 2.88 min (system 4); LC/MS MS: m/z = 373 (MH⁺), [α]_{Hg578} = +0.72° (c=0.09, DCM, 20 °C).

### Example 4.30: [5-Chloro-6-(6-methyl-pyridin-3-ylamino)-pyridin-3-yl]-((S)-2-isopropyl-piperidin-1-yl)-methanone

TLC: R_{f} = 0.63 (DCM/MeOH 5:1), HPLC: t_{R} = 2.89 min (system 4); LC/MS MS: m/z = 373 (MH⁺), [α]_{Hg578} = -0.79° (c=0.09, DCM, 20 °C).

### Example 4.31: [5-Chloro-6-(6-methyl-pyridin-3-ylamino)-pyridin-3-yl]-((R)-3-ethyl-piperidin-1-yl)-methanone

TLC: R_{f} = 0.30 (EtOAc/hexanes 1:1), HPLC: t_{R} = 2.83 min (system 4); LC/MS MS: m/z = 359 (MH⁺).

### Example 4.32: [5-Chloro-6-(6-methyl-pyridin-3-ylamino)-pyridin-3-yl]-((S)-3-ethyl-piperidin-1-yl)-methanone

TLC: R_{f} = 0.28 (EtOAc/hexanes 1:1), HPLC: t_{R} = 2.86 min (system 4); LC/MS MS: m/z = 359 (MH⁺).

### Example 4.33: [5-Chloro-6-(6-methyl-pyridin-3-ylamino)-pyridin-3-yl]-(3-cyclopropyl-piperidin-1-yl)-methanone

TLC: R_{f} = 0.51 (DCM/MeOH 5:1), HPLC: t_{R} = 2.90 min (system 4); LC/MS MS: m/z = 373 (MH⁺).

The starting material can be prepared as described hereafter:
i) *3-Cyclopropyl-piperidine hydrochloride*
   3-Cyclopropyl pyridine (820 mg, 5.27 mmol) was hydrogenated in a mixture of MeOH (15mL) and concentrated aqueous hydrochloric acid (0.58 mL) in the presence of Nishimura catalyst (70 mg) under atmospheric pressure for 22 hours. The mixture was filtered through a pad of celite and washed with MeOH. The solvent was removed in vacuo and the residue was dissolved in water. The aqueous solution was first washed with DCM, than basified by addition of 40% NaOH solution and extracted twice with DCM. The organic phases were combined, dried over sodium sulfate, acidified by addition of ethanolic hydrochloric acid, and concentrated in vacuo to afford 3-cyclopropyl piperidine hydrochloride (694 mg, 82%) as colorless crystals. TLC: R_{f} = 0.49 (DCM/MeOH 5:1), LC/MS MS: m/z = 126 (MH⁺).

### Example 4.34: [5-Chloro-6-(6-methyl-pyridin-3-ylamino)-pyridin-3-yl]-(2-propyl-piperidin-1-yl)-methanone

TLC: R_{f} = 0.84 (DCM/MeOH 5:1), HPLC: t_{R} = 2.95 min (system 4); LC/MS MS: m/z = 373 (MH⁺).

### Example 4.35: [5-Chloro-6-(6-methyl-pyridin-3-ylamino)-pyridin-3-yl]-((S)-2-propyl-piperidin-1-yl)-methanone

TLC: R_{f} = 0.69 (DCM/MeOH 5:1), HPLC: t_{R} = 2.97 min (system 4); LC/MS MS: m/z = 373 (MH⁺), [α]_{Hg578}= +1.17° (c=0.09, DCM, 20 °C).

### Example 4.36: [5-Chloro-6-(6-methyl-pyridin-3-ylamino)-pyridin-3-yl]-((R)-2-propyl-piperidin-1-yl)-methanone

TLC: R_{f} = 0.61 (DCM/MeOH 5:1), HPLC: t_{R} = 2.97 min (system 4); LC/MS MS: m/z = 373 (MH⁺), [α]_{Hg578}= -1.17° (c=0.09, DCM, 20 °C).

### Example 4.37: [5-Chloro-6-(6-methyl-pyridin-3-ylamino)-pyridin-3-yl]-(2,3-diethyl-piperidin-1-yl)-methanone

TLC: R_{f} = 0.21 (EtOAc/hexanes 1:1), HPLC: t_{R} = 3.08 min (system 4); LC/MS MS: m/z = 387 (MH⁺).

### Example 4.38: (2-Butyl-piperidin-1-yl)-[5-chloro-6-(6-methyl-pyridin-3-ylamino)-pyridin-3-yl]-methanone

TLC: R_{f} = 0.22 (DCM/MeOH 5:1), HPLC: t_{R} = 3.09 min (system 4); LC/MS MS: m/z = 387 (MH⁺).

### Example 4.39: [5-Chloro-6-(6-methyl-pyridin-3-ylamino)-pyridin-3-yl]-[2-(1-ethyl-propyl)-piperidin-1-yl]-methanone

TLC: R_{f} = 0.87 (DCM/MeOH 95:5), HPLC: t_{R} = 3.19 min (system 4); LC/MS MS: m/z = 401 (MH⁺).

### Example 4.40: [5-Chloro-6-(6-methyl-pyridin-3-ylamino)-pyridin-3-yl]-(2-ethyl-3-methyl-piperidin-1-yl)-methanone

TLC: R_{f} = 0.22 (EtOAc/hexanes 3:1), HPLC: t_{R} = 2.89 min (system 4); LC/MS MS: m/z = 373 (MH⁺).

The starting material was prepared as described hereafter:
*i) 2-Etyhl-3-methyl-pyridine*
   2-Ethyl-3-methylpyridine was prepared by Suzuki coupling of 2-bromo-3-methylpyridine and ethylboronic acid according to the procedure given in Tetrahedron Letters 2002, 43, 6987-6990. The desired product was obtained in 52% yield after purification on silica gel.
ii) *2-Etyhl-3-methyl-piperidine hydrochloride*
   2-Ethyl-3-methyl pyridine (1.75 g, 11.1 mmol) was hydrogenated in a mixture of MeOH (32 mL) and concentrated aqueous hydrochloric acid (1.2 mL) in the presence of Nishimura catalyst (180 mg) under atmospheric pressure for 22 hours. The mixture was filtered through a pad of celite and washed with MeOH. The solvent was removed in vacuo and the residue was dissolved in water. The aqueous solution was first washed with DCM, than basified by addition of 40% NaOH solution and extracted twice with DCM. The organic phases were combined, dried over sodium sulfate, acidified by addition of ethanolic hydrochloric acid, and concentrated in vacuo to afford 2-ethyl-3-methyl piperidine hydrochloride (1.60 g, 88%) as colorless crystals.

### Example 4.41: [5-Chloro-6-(6-methyl-pyridin-3-ylamino)-pyridin-3-yl]-(2-phenyl-piperidin-1-yl)-methanone

TLC: R_{f} = 0.34 (EtOAc/hexanes 3:1), HPLC: t_{R} = 1.85 min (system 5); LC/MS MS: m/z = 407 (MH⁺).

### Example 4.42: [5-Chloro-6-(6-methyl-pyridin-3-ylamino)-pyridin-3-yl]-(3,4,5,6-tetrahydro-2H-[2,2']bipyridinyl-1-yl)-methanone

TLC: R_{f} = 0.17 (EtOAc/hexanes 3:1), HPLC: t_{R} = 2.29 min (system 5); LC/MS MS: m/z = 408 (MH⁺).

### Example 4.43: [5-Chloro-6-(6-methyl-pyridin-3-ylamino)-pyridin-3-yl]-(3,4,5,6-tetrahydro-2H-[2,3']bipyridinyl-1-yl)-methanone

TLC: R_{f} = 0.25 (DCM/MeOH 9:1), HPLC: t_{R} = 2.11 min (system 5); LC/MS MS: m/z = 408 (MH⁺).

### Example 4.44: [5-Chloro-6-(6-methyl-pyridin-3-ylamino)-pyridin-3-yl]-(2-(tetrahydro-furan-2-yl)-piperidin-1-yl]-methanone

TLC: R_{f} = 0.34 (DCM/MeOH 9:1), HPLC: t_{R} = 2.62 min (system 4); LC/MS MS: m/z = 401 (MH⁺).

### Example 4.45: [5-Chloro-6-(6-methyl-pyridin-3-ylamino)-pyridin-3-yl]-[2-(5-methyl-furan-2-yl)-piperidin-1-yl]-methanone

TLC: R_{f} = 0.63 (DCM/MeOH 9:1), HPLC: t_{R} = 3.00 min (system 4); LC/MS MS: m/z = 411 (MH⁺).

### Example 4.46: [5-Chloro-6-(6-methyl-pyridin-3-ylamino)-pyridin-3-yl]-(2-oxazol-2-yl-piperidin-1-yl)-methanone

TLC: R_{f} = 0.55 (DCM/MeOH 9:1), HPLC: t_{R} = 2.66 min (system 4); LC/MS MS: m/z = 414 (MH⁺).

### Example 4.47: [2-(2-Chloro-ethyl)-piperidin-1-yl]-[5-chloro-6-(6-methyl-pyridin-3-ylamino)-pyridin-3-yl]-methanone

TLC: R_{f} = 0.33 (DCM/MeOH 5:1), HPLC: t_{R} = 0.76 min (system 4); LC/MS MS: m/z = 394 (MH⁺).

### Example 4.48: [5-Chloro-6-(6-methyl-pyridin-3-ylamino)-pyridin-3-ylj-(2,6-dimethyl-piperidin-1-yl)-methanone

TLC: R_{f} = 0.25 (EtOAc/hexanes 3:1), HPLC: t_{R} = 3.03 min (system 3); LC/MS MS: m/z = 359 (MH⁺).

### Example 4.49: [5-Chloro-6-(6-methyl-pyridin-3-ylamino)-pyridin-3-yl]-(2,2,6,6-tetramethyl-piperidin-1-yl)-methanone

TLC: R_{f} = 0.44 (EtOAc/hexanes 3:1), HPLC: t_{R} = 3.14 min (system 4); LC/MS MS: m/z = 387 (MH⁺).

### Example 4.50: [5-Chloro-6-(6-methyl-pyridin-3-ylamino)-pyridin-3-yl]-(2-methyl-6-ropyl-piperidin-1-yl)-methanone

TLC: R_{f} = 0.35 (EtOAc/hexanes 3:1), HPLC: t_{R} = 2.13 min (system 5); LC/MS MS: m/z = 387 (MH⁺).

### Example 4.51: [5-Chloro-6-(6-methyl-pyridin-3-ylamino)-pyridin-3-yl]-((2R,6R)-2-ethyl-6-propyl-piperidin-1-yl)-methanone

TLC: R_{f} = 0.45 (EtOAc/hexanes 3:1), HPLC: t_{R} = 2.25 min (system 5); LC/MS MS: m/z = 387 (MH⁺).

### Example 4.52: [5-Chloro-6-(6-methyl-pyridin-3-ylamino)-pyridin-3-yl]-(5-methyl-2-ropyl-piperidin-1-yl)-methanone

TLC: R_{f} = 0.40 (EtOAc/hexanes 3:1), HPLC: t_{R} = 2.06 min (system 5); LC/MS MS: m/z = 387 (MH⁺).

The starting material was prepared as described hereafter:
i) *5-Methyl-2-propyl-pyridine*
   5-Methyl-2-propyl-pyridine was prepared by Suzuki coupling of 2-bromo-5-methylpyridine and propylboronic acid according to the procedure given in Tetrahedron Letters 2002, 43, 6987-6990. The desired product was obtained in 24% yield after purification on silica gel.
ii) *5-Methyl-2-propyl-piperidine hydrochloride*
   5-Methyl-2-propyl-pyridine (345 mg, 2.55 mmol) was hydrogenated in a mixture of MeOH (10 mL) and concentrated aqueous hydrochloric acid (0.29 mL) in the presence of Nishimura catalyst (50 mg) under atmospheric pressure for 40 hours. The mixture was filtered through a pad of celite and washed with MeOH. The solvent was removed in vacuo and the residue was dissolved in water. The aqueous solution was first washed with DCM, than basified by addition of 40% NaOH solution and extracted twice with DCM. The organic phases were combined, dried over sodium sulfate, acidified by addition of ethanolic hydrochloric acid, and concentrated in vacuo to afford 2-methyl-3-propyl piperidine hydrochloride (0.43 g, 95%) as beige crystals.

### Example 4.53: [5-Chloro-6-(4-chloro-phenylamino)-pyridin-3-yl]-(octahydro-1]pyrindin-1-yl)-methanone

TLC: R_{f} = 0.71 (DCM/MeOH 95:5), HPLC: t_{R} = 3.85 min (system 5); LC/MS MS: m/z = 391 (MH⁺).

### Example 4.54: [5-Chloro-6-(4-chloro-phenylamino)-pyridin-3-yl]-((R)-2-ethyl-piperidin-1-yl)-methanone

TLC: R_{f} = 0.75 (DCM/MeOH 95:5), HPLC: t_{R} = 3.78 min (system 5); LC/MS MS: m/z = 379 (MH⁺).

### Example 4.55: [5-Chloro-6-(6-methyl-pyridin-3-ylamino)-pyridin-3-yl]-(2-vinyl-piperidin-1-yl)-methanone

TLC: R_{f} = 0.78 (DCM/MeOH 95:5), HPLC: t_{R} = 2.70 min (system 4); LC/MS MS: m/z = 357 (MH⁺).

### Example 4.56: [5-Chloro-6-(6-methyl-pyridin-3-ylamino)-pyridin-3-yl]-[((Z)-2-propenyl)-piperidin-1-yl]-methanone

TLC: R_{f} = 0.66 (DCM/MeOH 95:5), HPLC: t_{R} = 2.88 min (system 4); LC/MS MS: m/z = 371 (MH⁺).

### Example 4.57: [5-Chloro-6-(6-methyl-pyridin-3-ylamino)-pyridin-3-yl]-[3-ethylidene-piperidin-1-yl]-methanone

TLC: R_{f} = 0.89 (DCM/MeOH 5:1), HPLC: t_{R} = 2.85 min (system 4); LC/MS MS: m/z = 357 (MH⁺).

The starting material was prepared as described hereafter:
i) *3-Ethylidene-piperidine hydrochloride*
   To a solution of potassium-tert-butoxide (3.10 g, 27.6 mmol, 1.1 eq) in THF (30 mL) at rt was added sequentially ethyltriphenylphosphoniumbromide (11.0 g, 29.6 mmol, 1.18 eq) followed by a solution of 1-(tert-Butoxycarbonyl)-3-piperidone (5.0 g, 25.1 mmol) in THF (20 mL). After stirring the resulting suspension for 24 h at rt, water was added and the aqueous phase was extracted with DCM. The organic phases were combined, dried over sodium sulfate and the solvent removed on vacuo. After purification by flash chromatography, 3-ethylidene-piperidine-1-carboxylic acid tert-butyl ester (5.5 g, 100%) was obtained as a 1:2 E/Z isomeric mixture. Deprotection of the Boc-group was effected by stirring 3-ethylidene-piperidine-1-carboxylic acid tert-butyl ester (5.5 g, 26 mmol) in HCl/dioxane (4M, 15 mL) for 1 h at rt. The white precipitate was filtered off, washed twice with diethyl ether and dried on vacuo to afford the desired product as beige crystals (2.99 g, 78%). LC/MS MS: m/z = 111 (MH⁺).

### Example 4.58: [5-Chloro-6-(6-methyl-pyridin-3-ylamino)-pyridin-3-yl]-[3-propylidene-piperidin-1-yl]-methanone

TLC: R_{f} = 0.16 (DCM/MeOH 95:5), HPLC: t_{R} = 2.85 min (system 4); LC/MS MS: m/z = 371 (MH⁺).

3-Propylidene-piperidine hydrochloride was prepared in an overall yield of 71% starting from propyltriphenylphosphoniumbromide and 1-(tert-Butoxycarbonyl)-3-piperidone in analogy to the procedure given in Example 4.57 i. LC/MS MS: m/z = 126 (MH⁺).

### Example 4.59: [5-Chloro-6-(6-methyl-pyridin-3-ylamino)-pyridin-3-yl]-(2-ethoxymethyl-piperidin-1-yl)-methanone

TLC: R_{f} = 0.66 (DCM/MeOH 95:5), HPLC: t_{R} = 2.88 min (system 4); LC/MS MS: m/z = 371 (MH⁺).

### Example 4.60: [5-Chloro-6-(6-methyl-pyridin-3-ylamino)-pyridin-3-yl]-(2-thoxymethyl-piperidin-1-yl)-methanone

TLC: R_{f} = 0.26 (DCM/MeOH 95:5), HPLC: t_{R} = 2.55 min (system 4); LC/MS MS: m/z = 389 (MH⁺).

### Example 4.61: [5-Chloro-6-(6-methyl-pyridin-3-ylamino)-pyridin-3-yl]-[2-(2-hydroxy-ethyl)-piperidin-1-yl]-methanone

TLC: R_{f} = 0.23 (DCM/MeOH 95:5), HPLC: t_{R} = 2.72 min (system 4); LC/MS MS: m/z = 375 (MH⁺).

### Example 4.62: [5-Chloro-6-(6-methyl-pyridin-3-ylamino)-pyridin-3-yl]-(5-fluoro-2-propyl-piperidin-1-yl)-methanone

TLC: R_{f} = 0.35 (EtOAc/hexanes 2:1), HPLC: t_{R} = 1.60 min (system 5); LC/MS MS: m/z = 391 (MH⁺).

The starting material was prepared as described hereafter:
*i) 5-Fluoro-2-propyl pyridine*
   To a suspension of n-propylmagnesium chloride (13 mL, 1.0 M in diethyl ether) and zinc chloride (17 mL, 0.5 M in THF, 2.5 eq) was added 1-methyl-2-pyrrolidinone (10 mL), 2-bromo-5-fluorpyridine (600 mg, 3.41 mmol) and bis(tri-tert.-butylphosphine)palladium (174 mg, 0.34 mmol, 0.1 eq). After stirring at 80 °C for 3 h the mixture was cooled to 0°C, water was added resulting solution extracted with EtOAc twice. The organic phases were combined, dried over sodium sulfate and the solvent removed on vacuo. After purification by flash chromatography, 5-fluoro-2-propyl pyridine (182 mg, 30%) was obtained. LC/MS MS: m/z = 140 (MH⁺).
ii) *5-Fluoro-2-propyl-piperidine hydrochloride*
   5-Fluoro-2-propyl pyridine (182 mg, 1.04 mmol) was hydrogenated in a mixture of MeOH (10 mL) and concentrated aqueous hydrochloric acid (0.13 mL) in the presence of Nishimura catalyst (50 mg) at 4 bar for 3.5 hours. The mixture was filtered through a pad of celite and washed with MeOH. The solvent was removed in vacuo and the residue was dissolved in water. The aqueous solution was first washed with DCM, than basified by addition of 40% NaOH solution and extracted twice with DCM. The organic phases were combined, dried over sodium sulfate, acidified by addition of ethanolic hydrochloric acid, and concentrated in vacuo to afford a mixture of 5-fluoro-2-propyl-piperidine hydrochloride and 2-propyl-piperidine hydrochloride as light red solid (95%) which was used in the next step without further purification.

### Example 4.63: [5-Chloro-6-(6-methyl-pyridin-3-ylamino)-pyridin-3-yl]-[2-(1,2-difluoro-propyl)-piperidin-1-yl]-methanone and Example 4.64: [5-Chloro-6-(6-methyl-pyridin-3-ylamino)-pyridin-3-yl]-[2-(2-fluoro-propyl)-piperidin-1-yl]-methanone

Both compounds were isolated after preparative TLC separation of the corresponding mixture.

*[5-Chloro-6-(6-methyl-pyridin-3-ylamino)-pyridin-3-yl]-[2-(1,2-difluoro-propyl)-piperidin-1-yl]-methanone:* TLC: R_{f} = 0.39 (EtOAc/hexanes 5:1), HPLC: t_{R} = 1.37 min (system 5); LC/MS MS: m/z = 391 (MH⁺).

*[5-Chloro-6-(6-methyl-pyridin-3-ylamino)-pyridin-3-yl]-[2-(1-fluoro-propyl)-piperidin-1-yl]-methanone:* TLC: R_{f} = 0.40 (EtOAc/hexanes 5:1), HPLC: t_{R} = 1.22 min (system 5); LC/MS MS: m/z = 409 (MH⁺).

The starting material was prepared as described hereafter:
i) 2-(1,2-Difluoro-propenyl)-pyridine
   To a solution of 1-pyridin-2-yl-propan-2-one (3.75 g, 27.7 mmol) in DCM (20 mL) at 0°C was was DAST (10.1 mL, 69 mmol, 2.50 eq). After stirring the solution for 15 h (0°C→rt) it was diluted by DCM and subsequently quenched by slow addition of ice water. Resulting solution was extracted twice with DCM. The organic phases were combined, dried over sodium sulfate and the solvent removed on vacuo. After purification by flash chromatography 2-(1,2-difluoro-propenyl)-pyridine (616 mg, 14%) was obtained as beige oil. LC/MS MS: m/z = 156 (MH⁺).
ii) *2-(1,2-Difluoro-propyl)-piperidine hydrochloride, 2-(1-fluoro-propyl)-piperidine hydrochloride* and *2-propyl-piperidine hydrochloride*
   2-(1,2-Difluoro-propenyl)-pyridine (820 mg, 4.28 mmol) was hydrogenated in a mixture of MeOH (25 mL) and concentrated aqueous hydrochloric acid (0.46 mL) in the presence of Nishimura catalyst (100 mg) at atmospheric pressure for 24 hours. The mixture was filtered through a pad of celite and washed with MeOH. The solvent was removed in vacuo and the residue was dissolved in water. The aqueous solution was first washed with DCM, than basified by addition of 40% NaOH solution and extracted twice with DCM. The organic phases were combined, dried over sodium sulfate, acidified by addition of ethanolic hydrochloric acid, and concentrated in vacuo to afford a mixture of 2-(1,2-difluoro-propyl)-piperidine hydrochloride, 2-(1-fluoro-propyl)-piperidine hydrochloride and 2-propyl-piperidine hydrochloride as light red solid (100%) which was used in the next step without further purification.

### Example 4.65: [5-Chloro-6-(6-methyl-pyridin-3-ylamino)-pyridin-3-yl]-(2-ethyl-[1,3]o xazepan-3-yl)-methanone

TLC: R_{f} = 0.21 (DCM/MeOH 95:5), HPLC: t_{R} = 2.66 min (system 4); LC/MS MS: m/z = 375 (MH⁺).

### Example 4.66: [5-Chloro-6-(6-methyl-pyridin-3-ylamino)-pyridin-3-yl]-(2-propyl-[1,3]oxazepan-3-yl)-methanone

TLC: R_{f} = 0.2 (DCM/MeOH 95:5), HPLC: t_{R} = 2.82 min (system 4); LC/MS MS: m/z = 389 (MH⁺).

### Example 4.67: [5-Chloro-6-(6-methyl-pyridin-3-ylamino)-pyridin-3-yl]-[2-(1-ethyl-propyl)-[1,3]oxazepan-3-yl]-methanone

TLC: R_{f} = 0.17 (DCM/MeOH 95:5), HPLC: t_{R} = 3.10 min (system 4); LC/MS MS: m/z = 417 (MH⁺).

### Example 4.68: [5-Chloro-6-(6-methyl-pyridin-3-ylamino)-pyridin-3-yl]-(2-propyl-[1,3]oxazinan-3-yl)-methanone

TLC: R_{f} = 0.11 (DCM/MeOH 95:5), HPLC: t_{R} = 2.55 min (system 4); LC/MS MS: m/z = 375 (MH⁺).

### Example 4.69: [5-Chloro-6-(6-methyl-pyridin-3-ylamino)-pyridin-3-yl]-[2-(1-ethyl-propyl)-[1,3]oxazinan-3-yl]-methanone

TLC: R_{f} = 0.18 (DCM/MeOH 95:5), HPLC: t_{R} = 2.89 min (system 4); LC/MS MS: m/z = 403 (MH⁺).

### Example 4.70: (2-Butyl-[1,3]oxazinan-3-yl)-[5-chloro-6-(6-methyl-pyridin-3-ylamino)-pyridin-3-yl]-methanone

TLC: R_{f} = 0.12 (DCM/MeOH 95:5), HPLC: t_{R} = 2.87 min (system 4); LC/MS MS: m/z = 389 (MH⁺).

### Example 4.71: [5-Chloro-6-(6-methyl-pyridin-3-ylamino)-pyridin-3-yl]-[2-(2-methoxy-ethyl)-piperidin-1-yl]-methanone

TLC: R_{f} = 0.15 (DCM/MeOH 95:5), HPLC: t_{R} = 2.60 min (system 4); LC/MS MS: m/z = 389 (MH⁺).

### Example 4.72: [5-Chloro-6-(6-methyl-pyridin-3-ylamino)-pyridin-3-yl]-(2-phenyl-pyrrolidin-1-yl)-methanone

TLC: R_{f} = 0.66 (DCM/MeOH 9:1), HPLC: t_{R} = 3.07 min (system 3); LC/MS MS: m/z = 393 (MH⁺).

### Example 4.73: [5-Chloro-6-(6-methyl-pyridin-3-ylamino)-pyridin-3-yl]-(2-pyridin-2-yl-pyrrolidin-1-yl)-methanone

TLC: R_{f} = 0.65 (DCM/MeOH 9:1), HPLC: t_{R} = 2.48 min (system 3); LC/MS MS: m/z = 394 (MH⁺).

### Example 4.74: [5-Chloro-6-(6-methyl-pyridin-3-ylamino)-pyridin-3-yl]-((R)-2-ethoxy-pyrrolidin-1-yl)-methanone

TLC: R_{f} = 0.44 (DCM/MeOH 9:1), HPLC: t_{R} = 2.09 min (system 3); LC/MS MS: m/z = 361 (MH⁺).

### Example 4.75: [5-Chloro-6-(6-methyl-pyridin-3-ylamino)-pyridin-3-yl]-[2-(5-methyl-thiophen-2-yl)-pyrrolidin-1-yl]-methanone

TLC: R_{f} = 0.5 (DCM/MeOH 9:1), HPLC: t_{R} = 3.15 min (system 3); LC/MS MS: m/z = 413 (MH⁺).

### Example 4.76: [5-Chloro-6-(6-methyl-pyridin-3-ylamino)-pyridin-3-yl]-(2-propyl-azepan-1-yl)-methanone

TLC: R_{f} = 0.52 (DCM/MeOH 95:5), HPLC: t_{R} = 3.21 min (system 3); LC/MS MS: m/z = 387 (MH⁺).

### Example 4.77: [5-Chloro-6-(6-methyl-pyridin-3-ylamino)-pyridin-3-yl]-(3-propyl-morpholin-4-yl)-methanone

TLC: R_{f} = 0.18 (DCM/MeOH 95:5), HPLC: t_{R} = 2.53 min (system 4); LC/MS MS: m/z = 375 (MH⁺).

### Example 5.1: Azepan-1-yl-[5-chloro-6-(4-chloro-phenylamino)-pyridin-3-yl]-methanone

To a solution of 5-Chloro-6-(4-chloro-phenylamino)-nicotinic acid (72 mg, 0.25 mmol) and DIPEA (67 µL, 0.38 mmol) in 1,2-dimethoxyethane (1.2 mL) is added HATU (97 mg, 0.25 mmol) in one portion. The reaction mixture is stirred for 30 min at RT. Then, hexamethylene imine (24 µL, 0.2 mmol) is injected and stirring is continued for further 6h. The reaction mixture is evaporated to dryness and the residue is purified by preparative HPLC (YMC Pack Pro C₁₈ 5 µm, 150 x 30 mm; AcN-H₂O-0.1% TFA gradient 10% -> 100% AcN; flow: 20 mL min⁻¹). The fractions containing the product are combined and acetonitrile is evaporated. The remaining aqueous solution is made alkaline by addition of solid NaHCO₃ and extracted with ethyl acetate. The organic layer is separated, washed with brine, dried over Na₂SO₄, and evaporated to dryness to afford the title compound as a colorless powder (75 mg, 81 %), HPLC: t_{R} = 7.0 min (system 1); ESI+ MS: m/z = 364.0, 366.0 (MH⁺).

The starting material can be prepared as described hereafter:

### 5-Chloro-6-(4-chloro-phenylamino)-nicotinic acid

A solution of 5,6-dichloronicotinic acid (0.5 g, 2.55 mmol) and 4-chloroaniline (293 mg, 2.30 mmol) in glacial acetic acid (5 mL) is microwave heated to 150 °C for 75 min. To the clear solution is added ethyl acetate (10 mL). After a short time the product starts to crystallize. The precipitate is filtered off, washed with ethyl acetate, and vacuum dried at room temperature to afford the desired product as a colorless powder (470 mg, 65%).

Following the same procedure, the following compounds can be prepared:

### Example 5.2: rac-[5-Chloro-6-(4-chloro-phenylamino)-pyridin-3-yl]-(3-methyl-piperidin-1-yl)-methanone

Colorless syrup, HPLC: t_{R} = 7.2 min (system 1); ESI+ MS: m/z = 364.0, 366.0 (MH⁺). Using either S-3-methylpiperidine or R-3-methylpiperidine as starting material the pure enantiomers could be prepared:

### Example 5.2a: [5-Chloro-6-(4-chloro-phenylamino)-pyridin-3-yl]-(S-3-methyl-piperidin-1-yl)-methanone

Brown gum, HPLC: t_{R} = 7.4 min (system 1); ESI+ MS: m/z = 364.0, 366.0 (MH⁺).

### Example 5.2b: [5-Chloro-6-(4-chloro-phenylamino)-pyridin-3-yl]-(R-3-methyl-piperidin-1-yl)-methanone

Brown gum, HPLC: t_{R} = 7.3 min (system 1); ESI+ MS: m/z = 364.0, 366.0 (MH⁺).

### Example 5.3: Azepan-1-yl-[2-(4-chloro-phenylamino)-pyrimidin-5-yl]-methanone

Colorless crystals, HPLC: t_{R} = 6.4 min (system 1); ESI+ MS: m/z = 331.5 (MH⁺).

### Example 5.4: [2-(4-Chloro-phenylamino)-pyrimidin-5-yl]-piperidin-1-yl-methanone Colorless crystals, HPLC: t_{R} = 6.2 min (system 1); ESI+ MS: m/z = 317.6 (MH⁺).

### Example 5.5: rac-[2-(4-Chloro-phenylamino)-pyrimidin-5-yl]-(3-methyl-piperidin-1-yl)-methanone

Colorless crystals, HPLC: t_{R} = 6.5 min (system 1); ESI+ MS: m/z = 331.6 (MH⁺).

### Example 6.1: Azepan-1-yl-[6-(4-chloro-phenylamino)-5-methoxy-pyridin-3-yl]-methanone

To a solution of azepan-1-yl-(6-chloro-5-methoxy-pyridin-3-yl)-methanone (198 mg, 0.70 mmol) and 4-chloroaniline (270 mg, 2.11 mmol) in toluene (5 mL) is added finely ground anhydrous K₂CO₃ (491 mg, 3.52 mmol). To the suspension obtained is added a still warm solution prepared by dissolving palladium(II) acetate (10 mg, 0.04 mmol) and BINAP (27 mg, 0.04 mmol) in toluene (1 mL) with stirring for 20 min at 90°C. The reaction mixture is stirred under argon for 21 h at 80 °C. After cooling ethyl acetate (40 mL) is added and the solution is extracted with water (3x 15 mL). The organic layer is isolated, dried over Na₂SO₄ and evaporated to dryness to give a dark green oil. The crude product is purified by flash chromatography (24 g silica gel, MeOH-MTBE gradient 2% -> 15% MeOH, flow 20 mL min¹). Recrytallization from Et₂O gives the desired compound as beige crystals, TLC: R_{f} = 0.14 (MTBE), HPLC: t_{R} = 7.0 min (system 1); ESI+ MS: m/z = 360.1 (MH⁺).

The starting material can be prepared as described hereafter:
*i) 6-chloro-5-methoxynicotinic acid*
   To a solution of methyl 6-chloro-5-hydroxynicotinate (0.95 g, 5.07 mmol, prepared according to WO 00/51614) in DMSO (9.5 mL) is added powdered 85% KOH (0.67 g, 10.1 mmol) followed by slow injection of methyl iodide (0.35 mL, 5.57 mmol). The reaction mixture is stirred over night at RT. To achieve complete hydrolysis of the intermediate ester water (1 mL) is added and stirring is continued for further 30 min. The solution is diluted with 1M HCl (100 mL) and extracted with ethyl acetate (1x 100 mL, 3x 50 mL). The combined organic extracts are dried over Na₂SO₄ and evaporated to give a yellow solid residue. Trituration with H₂O (20 mL) followed by drying *in* vacuo at 65°C affords the title compound as beige powder (846 mg, 89%).
*ii) Azepan-1-yl-(6-chloro-5-methoxy-pyridin-3-yl)-mefhanone*
   A mixture of 6-chloro-5-methoxynicotinic acid (272 mg, 1.45 mmol) and thionyl chloride (3.2 mL) is stirred for 30 min at 75 °C. The clear solution is evaporated to dryness and the residue is redissolved in DCM (4 mL) under argon. After the addition of triethylamin (2 mL, 14.5 mmol) and hexamethyleneamine (0.2 mL, 1.74 mmol) the yellow turbid reaction mixture is stirred for 1 h at RT. Then MTBE (30 mL) is added and the solution is extracted with H₂O (3x 10 mL), dried over Na₂SO₄ and evaporated to give the title compound as a yellow oil (407 mg, 100%). The material can be used in the next step without further purification.

Following the same procedure, the following compounds can be obtained:

### Example 6.2: Azepan-1-yl-[5-methoxy-6-(6-methyl-pyridin-3-ylamino)-pyridin-3-yl]-methanone]

Yellow lyophilisate, TLC: R_{f} = 0.16 (MTBE-MeOH 9:1), HPLC: t_{R} = 4.5 min (system 1); ESI+ MS: m/z = 341.1 (MH⁺).

### Example 6.3: [6-(4-Chloro-phenylamino)-5-methoxy-pyridin-3-yl]-piperidin-1-yl-methanone

Yellowish crystals, TLC: R_{f} = 0.13 (MTBE), HPLC: t_{R} = 6.9 min (system 1); ESI+ MS: m/z = 346.1 (MH⁺).

### Example 6.4: [5-Methoxy-6-(6-methyl-pyridin-3-ylamino)-pyridin-3-yl]-piperidin-1-yl-methanone

Yellowish lyophilisate, TLC: R_{f} = 0.15 (MTBE-MeOH 9:1), HPLC: t_{R} = 4.0 min (system 1); ESI+ MS: m/z = 327.1 (MH⁺).

### Example 6.5: Azepan-1-yl-[6-(4-chloro-phenylamino)-5-ethoxy-pyridin-3-yl]-methanone

Colorless lyophilisate, TLC: R_{f} = 0.29 (MTBE), HPLC: t_{R} = 7.4 min (system 1); ESI+ MS: m/z = 374.1 (MH⁺).

### Example 6.6: Azepan-1-yl-[5-ethoxy-6-(6-methyl-pyridin-3-ylamino)-pyridin-3-yl]-methanone

Colorless lyophilisate, TLC: R_{f} = 0.25 (MTBE-MeOH 9:1), HPLC: t_{R} = 4.6 min (system 1); ESI+ MS: m/z = 355.2 (MH⁺).

### Example 6.7: [5-Ethoxy-6-(6-methyl-pyridin-3-ylamino)-pyridin-3-yl]-piperidin-1-yl-methanone

Colorless lyophilisate, TLC: R_{f} = 0.21 (MTBE-MeOH 9:1), HPLC: t_{R} = 4.4 min (system 1); ESI+ MS: m/z = 341.2 (MH⁺).

### Example 7.1: [5-Chloro-6-(6-chloro-pyridin-3-ylamino)-pyridin-3-yl]-(3-methyl-piperidin-1-yl)-methanone

A solution of 5-[3-Chloro-5-(3-methyl-piperidine-1-carbonyl)-pyridin-2-ylamino]-1H-pyridin-2-one (88 mg, 0.25 mmol) and DMAP (5 mg, 0.04 mmol) in phosphoryl chloride (2.75 mL) is refluxed under argon for 90 h. After cooling the suspension obtained is evaporated and taken up in DCM (40 mL) - 20% KHCO₃ solution (40 mL). The organic layer is washed (1x 20% KHCO₃, 40 mL; 2x H₂O, 20 mL), dried over Na₂SO₄ and evaporated to give a reddish turbid syrup. The crude material is purified by flash chromatography (25 g silica gel, eluent MTBE, flow 20 mL min⁻) to afford a bluish foam (37 mg, 40%), TLC: R_{f} = 0.39 (MTBE), HPLC: t_{R} = 6.4 min (system 1); ESI+ MS: m/z = 365.0 (MH⁺).

The starting material can be prepared as described hereafter:

### 5-[3-Chloro-5-(3-methyl-piperidine-1-carbonyl)-pyridin-2-ylamino]-1H-pyridin-2-one

To a solution of [5-Chloro-6-(6-methoxy-pyridin-3-ylamino)-pyridin-3-yl]-(3-methyl-piperidin-1-yl)-methanone (489 mg, 1.36 mmol, prepared from 5,6-dichloronicotinic acid, 3-methylpiperidine and 3-amino-6-methoxypyridine according to the procedure given in example 5.1) in 1,2-dichloroethane (30 mL) is added iodotrimethyl silane (0.47 mL, 3.39 mmol) in one portion. The reaction mixture is stirred for 6 h at 70°C under argon. After cooling the reaction is quenched with methanol (3 mL) stirred for 15 min at RT and evaporated. The residue is taken up in a mixture of DCM (40 mL) and triethyl amine (1 mL), extracted (1x H₂O, 20 mL; 1x 5% NaS₂O₃, 20 mL, 1x H₂O, 20 mL), dried over Na₂SO₄ and evaporated to afford greenish residue. The crude product is purified by flash chromatography (54 g silica gel, MeOH-DCM gradient 0% -> 10% MeOH, flow 40 mL min⁻¹) to afford a beige foam (401 mg, 85%).

### Example 8.1: [6-(4-Chloro-phenylamino)-pyridazin-3-yl]-piperidin-1-yl-methanone

To a solution of 6-(4-Chloro-phenylamino)-pyridazine-3-carboxylic acid (50 mg, 0.2 mmol) and DIPEA (53 µL, 0.3 mmol) in DMA (1 mL) is added HATU (76 mg, 0.2 mmol) in one portion. The reaction mixture is stirred for 30 min at RT. Then piperidine (16 uL, 0.16 mmol) is injected and stirring is continued for further 6 h. The solution is diluted with ethyl acetate (20 mL), extracted (2x brine, 20 mL), dried over Na₂SO₄ and evaporated to dryness to give an olive solid. The crude product is purified by flash chromatography (10 g silica gel, ETOAC-hexanes gradient 0% -> 80% ETOAC, flow 15 mL min⁻¹) followed by crystallization from ether / hexanes to afford the title compound as beige powder (21 mg, 33%), HPLC: t_{R} = 5.8 min (system 1); ESI+ MS: m/z = 317.5 (MH⁺).

The starting material can be prepared as described hereafter:

### 6-(4-Chloro-phenylamino)-pyridazine-3-carboxylic acid

A solution of 6-chloropyridazine-3-carboxylic acid (0.5 g, 3.15 mmol, [5096-73-1]) and 4-chloroaniline (805 mg, 6.31 mmol) in 1,2-dimethoxyethane (5 mL) is microwave heated for 20 min at 100 °C. After cooling the reaction mixture is diluted with ethyl acetate (10 mL) and stirred for 5 min. The brown precipitate is filtered off and triturated with cold water (30 mL). The light brown suspension is filtered and washed with water. After vacuum dry at 45°C the product is obtained as a beige powder (250 mg, 32%).

Following the same procedure, the following compounds can be obtained:

### Example 8.2: rac-[6-(4-Chloro-phenylamino)-pyridazin-3-yl]-(3-methyl-piperidin-1-yl)-methanone

Gray powder, HPLC: t_{R} = 6.1 min (system 1); ESI+ MS: m/z = 331.6 (MH⁺).

### Example 8.3: [6-(4-Chloro-phenylamino)-pyridazin-3-yl]-(3,3-dimethyl-piperidin-1-yl)-methanone

Beige powder, HPLC: t_{R} = 6.3 min (system 1); ESI+ MS: m/z = 345.6 (MH⁺).

### Example 8.4: [6-(4-Chloro-phenylamino)-pyridazin-3-yl]-(3,4-dihydro-1H-isoquinolin-2-yl)-methanone

Beige powder, HPLC: t_{R} = 6.4 min (system 1); ESI+ MS: m/z = 365.6 (MH⁺).

### Example 8.5: [6-(4-Chloro-phenylamino)-pyridazin-3-yl]-(4-methyl-piperidin-1-yl)-methanone

Gray powder, HPLC: t_{R} = 6.1 min (system 1); ESI+ MS: m/z = 331.6 (MH⁺).

### Example 9.1: [5-Methyl-6-(6-methyl-pyridin-3-ylamino)-pyridin-3-yl]-piperidin-1-yl-methanone

To a solution of 5-methyl-6-(6-methyl-pyridin-3-ylamino)-nicotinic acid (130 mg, 0.534 mmol) in DMF (15 mL), HOBt (106 mg, 0.74 mmol) and 4-methylmorpholine (180 µL, 1.61 mmol) were added. After 10 min of stirring, EDC (146 mg, 0.74 mmol) and piperidine (74.6 µL, 0.74 mmol) were added and the resulting mixture was stirred at 50°C for 16 hours. The solvent was removed in vacuo and EtOAc was added. The organic phase was washed with a saturated solution of NaHCO₃, dried over sodium sulfate and concentrated in vacuo to afford a brown resin. The crude product was purified by flash chromatography over silica gel using EtOAc as solvent to afford [5-methyl-6-(6-methyl-pyridin-3-ylamino)-pyridin-3-yl]-piperidin-1-yl-methanone (30 mg, 18%) as a yellow resin.

The starting material was prepared as described hereafter:
i) N-tert-butyldimethylsilyl isopropyl formimidate
   At -40°C, to a suspension of isopropyl formimidate hydrochloride (12.9 g, 105 mmol) in DCM (150 mL), triethylamine (32.3 mL, 231 mmol) was added in once. Then, a solution of tert-butyldimethylsilyl triflate (24.6 mL, 105 mmol) in DCM (100 mL) was added drop-wise with keeping the temperature below -40°C. At the end of the addition, 25 mL of hexanes was added at once and the mixture was then allowed to reach RT. The precipitate was filtered off and washed with hexanes and DCM. The filtrate was concentrated in vacuo to afford a yellow paste. Et₂O was added and the residual triethylammonium triflate was removed by decantation. The etheral phase was concentrated in vacuo to afford N-tert-butyldimethylsilyl isopropyl formimidate as a clear oil (15.53 g, 73.5%) which will be used without further purification.
ii) 6-Hydroxy-5-methyl-nicotinic acid ethyl ester
   At RT, a solution of propionyl chloride (1.55 mL, 17.4 mmol) in 3.5 mL of toluene was added drop-wise to a solution of N-tert-butyldimethylsilyl isopropyl formimidate (3.51 g, 17.4 mmol) and triethylamine (12.2 mL, 87 mmol) in 10 mL of toluene. The resulting mixture was stirred at RT for 2 hours and then 10 mL of hexanes was added. The precipitate was removed by filtration and washed with hexanes (3 x 5 mL). The solution was concentrated in vacuo to afford a clear oil. This oil was solubilised in toluene (15 mL) and ethyl propiolate (1.2 mL, 11.6 mmol) was added. The resulting mixture was stirred at 85°C for 70 hours. The mixture was concentrated in vacuo and then diluted with HCl 2N. The aqueous phase was extracted with DCM. The organic phases were combined, dried over sodium sulfate and concentrated in vacuo to afford a crude yellow paste (3.5 g). The crude product was purified by flash chromatography over silica gel using Hexanes/EtOAc (75/25 to 0/100) as solvent gradient to afford 6-hydroxy-5-methyl-nicotinic acid ethyl ester (1.65 g, 78.5%) as a yellow powder. (ES-MS: m/z 182.1 [M+H]⁺, t_{R} 3.28 min (system 2)).
iii) 6-Chloro-5-methyl-nicotinic acid ethyl ester
   A mixture of 6-hydroxy-5-methyl-nicotinic acid ethyl ester (1.65 g, 9.11 mmol) in POCl₃ (2.55 mL, 27.3 mmol) was stirred at 120°C for 1.5 hour. The mixture was cooled down and poured into ice. The resulting precipitate was filtered off, washed with water and then solubilised in DCM. The organic phase was dried over sodium sulfate and then concentrated in vacuo to afford 6-chloro-5-methyl-nicotinic acid ethyl ester (1.55 g, 85%) as a dark brown solid. (ES-MS: *m*/*z* 241.1/243.1 [M+CH₃CN+H]⁺, t_{R} 5.12 min (system 2)).
iv) 5-Methyl-6-(6-methyl-pyridin-3-ylamino)-nicotinic acid ethyl ester
   A mixture of 6-chloro-5-methyl-nicotinic acid ethyl ester (750 mg, 3.76 mmol), 3-amino-6-methyl pyridine (609 mg, 5.64 mmol), Pd(OAc)₂ (26 mg, 0.11 mmol), rac-BINAP (72 mg, 0.11 mmol) and potassium carbonate (2.62 g, 18.8 mmol) in degassed toluene (20 mL) was stirred, under argon, at 80°C for 4 hours. EtOAc was added and the organic phase was washed with water, dried over sodium sulfate and concentrated in vacuo to afford the 5-methyl-6-(6-methyl-pyridin-3-ylamino)-nicotinic acid ethyl ester (1.02 g, 100%) as a black solid. (ES-MS: m/z 272.2 [M+H]⁺, t_{R} 3.37 min (system 2)).
v) 5-Methyl-6-(6-methyl-pyridin-3-ylamino)-nicotinic acid
   To a solution of 5-methyl-6-(6-methyl-pyridin-3-ylamino)-nicotinic acid ethyl ester (1.02 g, 3.76 mmol) in THF/MeOH (1/1, 40 mL), NaOH 2N (3.8 mL, 7.6 mmol) was added. The mixture was stirred at RT for 16 hours. The solvent was removed in vacuo and the crude was diluted with water. The aqueous was acidified to pH 4-5 by addition of HCl 2N. The resulting precipitate was removed by filtration and dried under high-vacuum to afford 5-methyl-6-(6-methyl-pyridin-3-ylamino)-nicotinic acid (615 mg, 67%) as a beige solid. (ES-MS: m/z 244.1 [M+H]⁺, t_{R} 2.77 min (system 2)).

### Example 9.2: [5-Fluoro-6-(6-methyl-pyridin-3-ylamino)-pyridin-3-yl]-piperidin-1-yl-methanone [5-Fluoro-6-(6-methyl-pyridin-3-ylamino)-pyridin-3-yl]-piperidin-1-yl-methanone was prepared following the procedure described in example 9.1.

TLC: R_{f} = 0.14 (EtOAc/hexanes 1:9), LC/MS: m/z = 315 (MH⁺).

The starting material can be prepared as described in example 9.1.v) and iv) starting from 6-Chloro-5-fluoro-nicotinic acid methyl ester.

### Example 10: Example of Biological Testing.

Activity of the Example compounds of the present invention, non limiting to the other compounds of the invention but merely used by way of example, was examined by measurement of the inhibition of the glutamate induced elevation of intracellular Ca²⁺-concentration following similar methods than those described in L. P. Daggett et al., Neuropharm. Vol. 34, pages 871-886 (1995), P. J. Flor et al., J. Neurochem. Vol. 67, pages 58-63 (1996).

The table below represents percentages of inhibition of the glutamate induced elevation of intracellular Ca²⁺-concentration at a concentration of 10 µM.

| **Compound Number** | **mGluR5 Activity inh. at 10 µM [%]** | | **Compound Number** | **mGluR5 Activity inh. at 10 µM [%]** |
|---|---|---|---|---|
| 1.1 | 95 | | 1.21 | 57 |
| 1.2 | 93 | | 2.1 | 60 |
| 1.3 | 75 | | 2.2 | 46 |
| 1.4 | 37 | | 2.3 | 65 |
| 1.5 | 90 | | 2.4 | 100 |
| 1.6 | 96 | | 2.5 | 97 |
| 1.7 | 77 | | 2.6 | 98 |
| 1.8 | 54 | | 2.7 | 98 |
| 1.9 | 97 | | 2.8 | 96 |
| 1.10 | 94 | | 2.9 | 100 |
| 1.11 | 71 | | 2.10 | 98 |
| 1.12 | 93 | | 2.10a | 99 |
| 1.12a | 99 | | 2.10b | 99 |
| 1.12b | 100 | | 2.11 | 100 |
| 1.13 | 96 | | 2.12 | 100 |
| 1.14 | 94 | | 2.13 | 96 |
| 1.15 | 88 | | 2.14 | 97 |
| 1.16 | 54 | | 2.15 | 100 |
| 1.17 | 83 | | 2.16 | 97 |
| 1.18 | 32 | | 2.17 | 97 |
| 1.19 | 94 | | 2.18 | 100 |
| 1.20 | 95 | | 2.19 | 96 |
| 2.20 | 96 | | 4.27 | - |
| 2.21 | 98 | | 4.28 | 93 |
| 2.22 | 98 | | 4.29 | 86 |
| 2.23 | 100 | | 4.30 | 96 |
| 2.24 | 97 | | 4.31 | 100 |
| 3.1 | 94 | | 4.32 | 100 |
| 3.2 | 97 | | 4.33 | 96 |
| 3.3 | 90 | | 4.34 | 98 |
| 4.1 | 99 | | 4.35 | 85 |
| 4.2 | 97 | | 4.36 | - |
| 4.3 | 92 | | 4.37 | 94 |
| 4.4 | 98 | | 4.38 | 98 |
| 4.5 | 88 | | 4.39 | 96 |
| 4.6 | 86 | | 4.40 | 98 |
| 4.7 | 87 | | 4.41 | 96 |
| 4.8 | 94 | | 4.42 | 98 |
| 4.9 | 37 | | 4.43 | 100 |
| 4.10 | - | | 4.44 | 99 |
| 4.11 | 51 | | 4.45 | 90 |
| 4.12 | 96 | | 4.46 | 94 |
| 4.13 | 97 | | 4.47 | 97 |
| 4.13a | 97 | | 4.48 | 99 |
| 4.13b | 96 | | 4.49 | 66 |
| 4.14 | 86 | | 4.50 | 98 |
| 4.15 | 98 | | 4.51 | 96 |
| 4.16 | 81 | | 4.52 | 96 |
| 4.17 | 98 | | 4.53 | 100 |
| 4.18 | 97 | | 4.54 | 97 |
| 4.19 | 97 | | 4.55 | 96 |
| 4.20 | 100 | | 4.56 | 97 |
| 4.21 | 99 | | 4.57 | 97 |
| 4.22 | 98 | | 4.58 | 93 |
| 4.23 | 95 | | 4.59 | - |
| 4.24 | 92 | | 4.60 | 97 |
| 4.25 | 98 | | 4.61 | 79 |
| 4.26 | 93 | | 4.62 | 99 |
| 4.63 | 100 | | 5.3 | 87 |
| 4.64 | 99 | | 5.4 | 83 |
| 4.65 | 83 | | 5.5 | 96 |
| 4.66 | 91 | | 6.1 | 97 |
| 4.67 | 95 | | 6.2 | 100 |
| 4.68 | 99 | | 6.3 | 95 |
| 4.69 | 99 | | 6.4 | 98 |
| 4.70 | 96 | | 6.5 | 33 |
| 4.71 | 99 | | 6.6 | 54 |
| 4.72 | 97 | | 6.7 | 35 |
| 4.73 | 72 | | 7.1 | 98 |
| 4.74 | 92 | | 8.1 | 91 |
| 4.75 | 96 | | 8.2 | 100 |
| 4.76 | 92 | | 8.3 | 82 |
| 4.77 | 100 | | 8.4 | 89 |
| 5.1 | 98 | | 8.5 | 86 |
| 5.2 | 100 | | 9.1 | 97 |
| 5.2a | 100 | | 9.2 | 99 |
| 5.2b | 98 | | | |

### Example 11: Clinical Testing

The action of mGluR modulatos, e.g. mGluR anatagonists on cognition and cognitive disorders, as described herein may be conducted in the following way.

Firstly, it has been found through imaging techniques that the compounds of the present invention are able to penetrate the brain and bind to mGluR receptors, in particular mGluR5 receptors. Secondly, it has been observed that patients taking a compound, such as an mGluR modulators as described herein have shown an increase in cognition or the like.

Clinical testing of the compounds as mentioned herein may be conducted, for example, in one of the following study designs. The skilled person will of course realise that such studies are considered as guidelines and the certain aspects of the studies may be modified and redefined depending on the circumstance and environment, for example.

### Clinical Design 1: Improvement Trials

### Trial A: Normal Patient Population

A patient population, with a normal control is dosed once a day for a week or longer and cognition is tested. The test is designed to allow for improvement, I.e. that there is a measurable parameter increase. The patients are tested at the beginning and at the end of the dosage period and the results are compared and analysed.

### Trial B: Deficit population

A patient population with a cognitive deficit is dosed once a day for a week or longer and cognition is tested. The test is designed to allow for improvement, I.e. that there is a measurable parameter increase. The patients are tested at the beginning and at the end of the dosage period and the results are compared and analysed.

### Considerations for designing a trial

- When designing a trial, the skilled person will appreciate the need to protect both against floor and ceiling effects. In other words, the study designing should allow cognition to the measurably raised or lowered.
- Conditions that artificially impair cognition, are one way to test enhancement of cognition. Such conditions are, for example, sleep deprivation and pharmacological challenges.
- Placebo control is required for all trials.
- In assessing the data, evaluation of the likelihood of learning and practice effects from repeat assessments must be made. The likelihood of such effects contaminating the data to produce false positives should be taken in to account when designing the test, e.g. the tests should not be identical (e.g. commit the same list of words to memory) but designed to study the same mechanism of cognition. Other countermeasures may include single testing at the end of a trial only.

## Claims

1. An mGluR modulator for the treatment, prevention or delay of progression of cognitive dysfunction.

2. The mGluR modulator according to claim 1, wherein the modulator is an mGluR5 modulator.

3. The mGluR modulator according to claim 1 or claim 2, wherein the modulator is an mGluR antagonist.

4. The mGluR modulator according to claim 2 or claim 3, wherein the modulator is an mGluR5 antagonist.

5. The mGluR modulator according to any of claims 1 to 4, wherein the modulator is a compound of the formula (IV) or the formula (V): 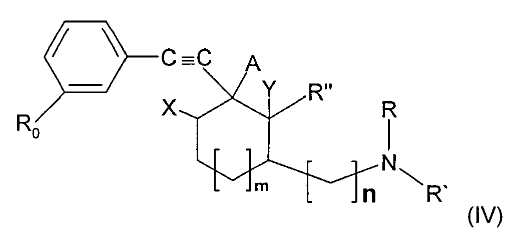 wherein
m is 0 or 1,
n is 0 or 1 and
A is hydroxy
X is hydrogen and
Y is hydrogen, or
A forms a single bond with X or with Y;
R₀ is hydrogen, (C₁₋₄)alkyl, (C₁₋₄)alkoxy, trifluoromethyl, halogen, cyano, nitro, -COOR, wherein R₁ is (C₁₋₄)alkyl or -COR₂ wherein R₂ is hydrogen or (C₁₋₄)alkyl, and
R is -COR₃, -COOR₃, -CONR₄R₅ or -SO₂R₆, wherein R₃ is (C₁₋₄)alkyl, (C₃₋₇)cycloalkyl or optionally substituted phenyl, 2-pyridyl or 2-thienyl; R₄ and R₅, independently, are hydrogen or (C₁₋₄)alkyl; and R₆ is (C₁₋₄)alkyl, (C₃₋₇)cycloalkyl or optionally substituted phenyl,
R' is hydrogen or (C₁₋₄)alkyl and
R" is hydrogen or (C₁₋₄)alkyl, or
R' and R" together form a group -CH₂-(CH₂)ₘ-
wherein m is 0, 1 or 2, in which case one of n and m is different from 0,
with the proviso that R₀ is different from hydrogen, trifluoromethyl and methoxy when n is 0, A is hydroxy, X and Y are both hydrogen, R is COOEt and R' and R" together form a group - (CH₂)₂-,
OR 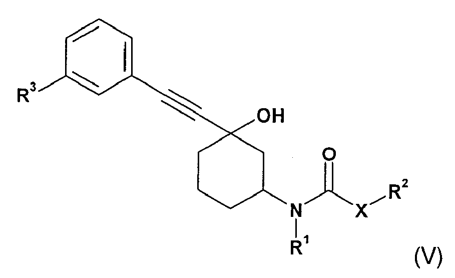 wherein
R¹ represents hydrogen or alkyl;
R² represents an unsubstituted or substituted heterocycle or
R² represents an unsubstituted or substituted aryl;
R³ represents alkyl or halogen;
X represents a single bond or an alkandiyl-group, optionally interrupted by one or more oxygen atoms or carbonyl groups or carbonyloxy groups;
in free base or acid addition salt form.

6. The mGluR modulator according to any preceding claim, wherein the cognitive dysfunction comprises a disorder selected from deficits and abnormalities in attention and vigilance, executive functions and memory (for instance working memory and episodic memory), sleep related breathing disorders (SRBD), behavioral impairments, information processing deficits and age-related disorders.

7. The mGluR modulator according to any preceding claim, wherein the cognitive dysfunction is associated with a disorder selected from sleep related breathing disorders (SRBDs), behavioral impairments, attention-deficit hyperactivity disorder (ADHD), childhood ADHD, adult ADHD, excess daytime somnolence, sleep apnea, shift-worker's sleep-wake cycle disruption, traumatic brain injury, neurodegenerative disorders with associated memory, cognitive problems (such as Alzheimer's disease, Lewy body dementia, senile dementia, vascular dementia, Parkinson's disease), chronic fatigue syndrome, fatigue associated with sleep deprivation or prolonged wakefulness, age-related decline in memory and cognitive function (such as mild cognitive impairment), mood disorders (such as depression), anxiety, schizophrenia and daytime sleepiness associated with narcolepsy.

8. A pharmaceutical composition comprising an mGluR modulator, for the treatment, prevention or delay of progression of cognitive dysfunction.

9. A composition according to claim 8, wherein the mGluR modulator is as defined in any of claims 2 to 5.

10. A composition according to claim 8 or claim 9, wherein the mGluR modulator is an mGluR5 modulator

11. A composition according to claim 9or claim 10, wherein the mGluR modulator is an mGluR5 antagonist.

12. A kit comprising an mGluR modulator and instructions for using the modulator in the treatment, prevention or delay of progression of cognitive dysfunction.
